# EUROPÄISCHE PATENTSCHRIFT

(11) **EP 0 678 505 B1**
(45) Veröffentlichungstag und Bekanntmachung des Hinweises auf die Patenterteilung: **04.06.1997**
(21) Anmeldenummer: 95110519.6
(22) Anmeldetag: 17.01.1992
(51) Int. Cl.: C07C 251/48, C07C 251/50, A01N 35/10, A01N 37/50

(54) **Oximether und diese enthaltende Fungizide**
Oximethers and fungicides containing them
Oximéthers et fongicides les contenant

(30) Priorität: 07.02.1991 DE 4103695
(43) Veröffentlichungstag der Anmeldung: 25.10.1995
(62) Teilanmeldung aus: 92100713.4
(73) Patentinhaber: BASF AKTIENGESELLSCHAFT, 67056 Ludwigshafen (DE)
(72) Erfinder: Benoit, Remy, Dr., D-67435 Neustadt (DE); Sauter, Hubert, Dr., D-68167 Mannheim (DE); Kirstgen, Reinhard, Dr., D-67434 Neustadt (DE); Lorenz, Gisela, Dr., D-67434 Hambach (DE); Ammermann, Eberhard, Dr., D-64646 Heppenheim (DE)

(56) Entgegenhaltungen:
- EP-A- 0 006 254
- EP-A- 0 254 426
- EP-A- 0 363 818
- EP-A- 0 407 891

## Beschreibung

Die vorliegende Erfindung betrifft neue Oximetherderivate, ihre Herstellung und ihre Verwendung als Fungizide.

Es ist bekannt, Oximether z.B. den 1-Methoxyimino-1-(2-phenoxymethylphenyl)-essigsäuremethylester als Fungizide zu verwenden (EP 253 213). Ihre Wirkung ist jedoch unbefriedigend.

Es wurde nun gefunden, daß neue Oximether der Formel I 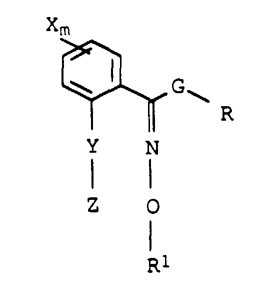 in der
- G: C=W, bedeutet,
- W: N-R⁵ bedeutet,
- R: gegebenenfalls substituiertes C₃-C₈-Cycloalkyl, gegebenenfalls substituiertes C₂-C₈-Alkenyl, gegebenenfalls substituiertes C₂-C₈-Alkinyl, gegebenenfalls substituiertes Phenyl, gegebenenfalls substituiertes Hetaryl oder 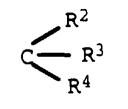 bedeutet,
- R¹: Wasserstoff, C₁-C₈-Alkyl bedeutet,
- R²: Wasserstoff, C₁-C₈-Alkyl bedeutet, Arylalkyl, Hetarylalkyl, Alkoxyalkyl, Aryloxyalkyl, Aryl, Hetaryl, bedeutet,
- R³: Wasserstoff, Methyl, Cyano, -COO-A bedeutet,
- A: Wasserstoff, C₁-C₈-Alkyl bedeutet,
oder
für den Fall, daß R³ den Rest -COO-C₁-C₈-Alkyl bedeutet, R² und R³ zusammen mit dem C, dessen Substituenten sie sind, einen 5- bis 8-gliedrigen Lactonring bedeuten,
- R⁴: Wasserstoff, C₁-C₈-Alkyl, Arylalkyl, Hetarylalkyl, Alkoxyalkyl, Aryloxyalkyl, Aryl, Hetaryl bedeutet,
oder
- R² und R⁴: zusammen mit dem C, dessen Substituenten sie sind, einen C₃-C₈-Cycloalkylring bedeuten,
- R⁵: Wasserstoff, C₁-C₈-Alkyl, C₁-C₄-Arylalkyl, C₃-C₆-Cycloalkyl, Hydroxy, C₁-C₄-Alkyloxy, NH₂, C₁-C₄-Alkylamino, C₁-C₄-Dialkylamino bedeutet,
- Y: gegebenenfalls substituiertes C₁-C₈-Alkylen, gegebenenfalls substituiertes C₂-C₈-Alkenylen, C₂-C₈-Alkinylen, O, S(O)ₘ (m = 0, 1, 2), N, das substituiert ist durch gegebenenfalls substituiertes C₁-C₈-Alkyl; Oxycarbonyl, Carbonyloxy, Oxycarbonyl-C₁-C₈-alkyl, Carbonyloxy-C₁-C₈-alkyl, C₂-C₈-Oxyalkyloxy, C₂-C₈-Oxyalkenyl, C₁-C₈-Alkyloxy, Oxy-C₁-C₄-alkyl, C₁-C₈-Thioalkyl, Azo, gegebenenfalls C₁-C₈-alkylsubstituiertes Carbonylamino, gegebenenfalls C₁-C₈-alkylsubstituiertes Aminocarbonyl, gegebenenfalls C₁-C₈-alkylsubstituiertes Aminocarbonyloxy, Oxyimino, gegebenenfalls substituiertes Iminooxyalkyl bedeutet,
- Z: C₁-C₁₈-Alkyl, C₂-C₁₈-Alkenyl, C₃-C₁₈-Alkinyl, Aryl, Aryl-C₁-C₁₀-Alkyl, Aryl-C₂-C₁₀-Alkenyl, Aryloxy-C₁-C₁₀-Alkyl, C₁-C₄-Alkoxy-C₁-C₁₀-alkyl, C₁-C₁₀-Halogenalkyl, C₁-C₄-Alkoxycarbonyl, Hetaryl bedeutet,

Z bedeutet für den Fall, daß Y nicht Oxyalkyl, Oxyalkyloxy, Thialkyl, -O-N=C-, -N=N-, Oxycarbonyl, Oxycarbonyloxy, O oder S ist, zusätzlich noch Aryloxy, Aryloxy-C₁-C₁₀-alkoxy, Aryl-C₁-C₁₀-alkoxy, C₁-C₁₀-Alkoxy oder Hetaryloxy,
Z bedeutet für den Fall, daß Y nicht Oxyalkyl, Oxyalkyloxy, -O-N=C-, -N=N-, Oxycarbonyl, Oxycarbonylalkyl oder O ist, zusätzlich noch C₁-C₁₈-Alkylthio oder Arylthio,
wobei die für Z genannten Reste gegebenenfalls substituiert sind durch Halogen, Cyano, Nitro, C₁-C₄-Alkyl, C₂-C₄-Alkenyl, C₁-C₄-Alkoxy, C₁-C₄-Halogenalkyl, C₁-C₄-Alkoxy-C₁-C₄-alkyl, C₁-C₄-Dialkoxy-C₁-C₄-Alkyl, gegebenenfalls substituiertes O-C₁-C₁₀-Alkyloxyimino, gegebenenfalls substituiertes O-C₂-C₁₀-Alkenyloxyimino, gegebenenfalls substituiertes O-Aralkyloxyimino, gegebenenfalls substituiertes C₂-C₁₀-Alkanoyl, gegebenenfalls substituiertes Formyl, C₂-C₄-Halogenalkenyl, C₁-C₄-Alkoxycarbonyl, gegebenenfalls substituiertes Phenyl, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten einen gegebenenfalls substituierten aromatischen oder heteroaromatischen Ring bilden können,
- X: gleich oder verschieden ist und Wasserstoff, Halogen, Nitro, gegebenenfalls substituiertes C₁-C₄-Alkyl, gegebenenfalls substituiertes C₁-C₄-Alkoxy oder gegebenenfalls substituiertes C₁-C₄-Halogenalkyl bedeutet und
- m: eine ganze Zahl zwischen 1 und 4 bedeutet
eine sehr gute fungizide Wirkung haben, die besser als die der bekannten Fungizide ist. Die neuen Verbindungen haben auch eine insektizide Wirkung. Die neuen Verbindungen der Formel I können bei ihrer Herstellung aufgrund der C==N-Doppelbindung als E-Z-Isomerengemische anfallen, die in üblicher Weise, z.B. durch Kristallisation oder Chromatographie, in die einzelnen Komponenten getrennt werden können. Sowohl die einzelnen isomeren Verbindungen als auch ihre Gemische werden von der Erfindung umfaßt und sind als Fungizide brauchbar.
- R: bedeutet bevorzugt Phenyl, Furyl, Thienyl, Cyclopropyl, Cyclobutyl, Cyclopentyl, Cyclohexyl, Vinyl oder 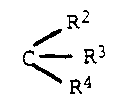
- R¹: bedeutet bevorzugt, Methyl, Ethyl, iso-Propyl, Propyl.
- R²: bedeutet bevorzugt Wasserstoff, Methyl, Ethyl, Propyl.
- R³: bedeutet bevorzugt Wasserstoff oder -CO-OCH₃.
- R⁴: bedeutet bevorzugt Wasserstoff, Methyl, Ethyl, Propyl.
- R⁵: bedeutet bevorzugt Methyl, Ethyl, n-Propyl, iso-Propyl, Cyclopropyl.
- Y: bedeutet bevorzugt Methylen, Ethylen, Ethenylen, Ethinylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C==N-O-CH₂-, -O-N==C-, -C==N-O, -N==N-Gruppe oder O oder S.
- Z: bedeutet bevorzugt Wasserstoff, Halogen (z.B. Fluor, Chlor, Brom), C₁-C₁₈-Alkyl (z.B. Methyl, Ethyl, n- und iso-Propyl, n-, sec-, iso- und tert.-Butyl, 3-Methylbutyl, 2,2-Dimethylpropyl, 1,1-Dimethylpropyl, 3-Hexyl, n-Pentyl, n-Hexyl, n-Heptyl, n-Octyl, n-NOnyl, n-Decyl, n-Pentadecyl, n-Heptadecyl, 2,6-Dimethylhept-l-yl); Cyanmethyl;
C₂-C₉-Alkenyl (z.B. Vinyl, 2-Propen-2-yl, 1-Propen-1-yl, 2-Buten-2-yl, 2-Methyl-prop-l-en-l-yl, Allyl, 2-Buten-1-yl, 3-Methyl-2-buten-1-yl, 1,3-Pentadien-1-yl, 2,6-Dimethyl-1,5-heptadien-1-yl, 2,6-Dimethyl-hept-5-en-1-yl); C₂-Alkinyl (z.B. Ethinyl, 2-Phenylethinyl);
C₁-C₂-Alkoxy-C₁-C₂-alkyl (Methoxymethyl, Ethoxymethyl, 1-Methoxyethyl), C₃-C₆-Cycloalkyl (z.B. Cyclopropyl, Cyclobutyl, Cyclopentyl, Cyclohexyl, 1-Methyl-cyclopropyl, wobei z.B. folgende substituierte Cyclopropylreste gemeint sind:
2,2-Dichlor-1-methyl-cyclopropyl (A1)
2,2-Dichlor-3,3-dimethyl-cyclopropyl (A2)
2,2,3,3-Tetramethylcyclopropyl (A3)
2-(2'-Methyl-1'-propenyl)-3,3-dimethylcyclopropyl (A4)
2-(2',2'-Difluorvinyl)-3,3-dimethylcyclopropyl (A5)
2-(2'-2'-Dichlorvinyl)-3-3-dimethylcyclopropyl (A6)
2-(2'-2'-Dibromvinyl)-3-3-dimethylcyclopropyl (A7)
2-Phenylcyclopropyl (A8)
2-(4'-Chlorphenyl)cyclopropyl (A9)
2,2-Dichlor-3-phenylcyclopropyl (A10)
und Halogen-C₁-C₂-alkyl (z.B. Chlormethyl, 1-Chlorethyl, Dichlormethyl, Trichlormethyl, Brommethyl, Trifluormethyl), Phenyl, substituiertes Phenyl, wie Halogenphenyl (z.B. 2-Fluor-, 3-Fluor-, 4-Fluor-, 6-Fluor-, 2-Chlor-, 2,4-Difluor-, 2,6-Difluor-, 2,3,4,5,6-Pentafluor-, 2-Chlor-, 3-Chlor-, 4-Chlor-, 2,5-Dichlor-, 2,5-Dichlor-, 2,6-Dichlor-, 3,4-Dichlor-, 3,5-Dichlor-, 2,4,5-Trichlor-, 2,3,4,5,6-Pentachlor-, 2-Brom-, 3-Brom-, 4-Bromphenyl); C₁-C₄-Alkylphenyl (z.B. 2-Methyl-, 3-Methyl-, 2,3-Dimethyl-, 2,4-Dimethyl-, 2,5-Dimethyl-, 2,6-Dimethyl-, 3,4-Dimethyl-, 3,5-Dimethyl-, 2,4,6-Trimethyl-, 4-t-Butyl-Phenyl); Arylphenyl (z.B. 2-Phenyl-, 4-Phenylphenyl); Halogen-C₁-alkylphenyl (z.B. 2-Trifluor-, 3-Trifluor-, 4-Trifluormethylphenyl); C₁-C₄-Alkoxyphenyl (z.B. 2-Methoxy-, 3-Methoxy-, 4-Methoxy-, 3,4-Dimethoxy-, 3,4,5-Trimethoxy-, 4-t-Butoxyphenyl); 2-, 3-, 4-Phenoxyphenyl); C₁-C₄-Alkanoylphenyl (z.B. 4-Acetyl-, 3-Acetyl-, 2-Acetyl-, 4-Acetyl-2-methylphenyl); Formylphenyl (z.B. 2-Formyl-, 3-Formyl-, 4-Formylphenyl); C₁-C₄-Dialkoxy-C₁-C₄-alkylphenyl [z.B. 4-(1,1-Dimethoxyethyl), 3-(Dimethoxymethyl), 4-(1,1-Dimethoxyethyl)-2-methylphenyl]; 3-(1,1-Dimethoxyethyl), 3-(Dimethoxymethyl), C₁-C₁₀-O-Alkyloxyiminophenyl (z.B. 4-[1-(O-Ethyloxyimino)ethyl], 4-(O-Ethyloxyiminomethyl), 3-[1-(O-Ethyloxyimino)ethyl]; 3-(O-Ethyloxyiminomethyl), 4-[1-(O-Ethyloxyimino)ethyl]-2-methyl, 4-[1-(O-Hexyloxyimino)ethyl], 4-(O-Hexyloxyiminomethyl), 3-[1-(O-Hexyloxyimino)ethyl], 3-(O-Hexyloxy-iminomethyl),4-[1-(O-Hexyloxyimino)ethyl]2-methyl, 4-[1-(O-iso-Butyloxyimino)ethyl], 4-(O-iso-Butyloxyiminomethyl), 3-[1-(O-iso-Butyloxyimino)-ethyl], 3-(O-iso-Butyloxyiminomethyl), 4-[1-(O-iso-Butyloxyimino)ethyl]-2-methylphenyl);
C₂-C₁₀-Alkenyloxyiminophenyl (z.B. 4-[1-(O-2-Buten-1-oxyimino)ethyl], 4-(O-2-Buten-1-oxyiminomethyl), 3-[1-(O-2-Buten-1-oxyimino)ethyl], 3-(O-2-Buten1-oxyiminomethyl), 4[1-(O-2-Buten-1-oxyimino)ethyl]-2-methylphenyl); C₁-C₁₀-Arylalkyloxyiminophenyl(z.B. 4-[1-(O-Benzyloxyimino)-ethyl], 4-(O-Benzyloxyiminomethyl), 3-[1-(O-Benzyloxyimino)ethyl], 3-(O-Benzyloxyiminomethyl), 4-[1-(O-Benzyloxyimino)ethyl]-2-methylphenyl, substituiertes Benzyl (z.B. Halogenbenzyl, 2-Fluor-, 3-Fluor-, 4-Fluor-, 2-Chlor-, 3-Chlor-, 4-Chlor-, 2-Chlor-, 6-Fluor-, 2,4-Dichlor-, 2,6-Dichlor-, 3,5-Dichlor-, 2,4,6-Trichlor-, 2-Brom-, 3-Brom-, 4-Brom-, 2-Methyl-, 3-Methyl-, 4-Methyl-, 4-t-Butyl-, 2,4-Dimethyl, 2,6-Dimethyl-, 2,4,6-Trimethyl-, 2-Methoxy-, 3-Methoxy-, 4-Methoxy-, 2-Trifluormethyl-, 3-Trifluormethyl-, 4-Trifluormethyl-, 4-t-Butoxy-, 4-Henoxy-, 4-Phenyl-benzyl, -Methyl-ethyl-, -iso-Propyl-, -Hydroxy-, 2-Methoxy-hydroxy-, 4-Methoxy-hydroxy-, 3,4-Dimethoxy-hydroxy-, 2-Methoxy-methoxy-, 4-Methoxy-methoxy-, 3,4-Dimethoxy-methoxybenzyl); gegebenenfalls substituiertes Phenylethyl (z.B. Phenyl-, 1-Methyl-2-phenyl-, 2-(para-t-Butyl-phenyl-), 2-(para-t-Butylphenyl)-1-Methyl-, 2-(ortho-Chlorphenyl)-, 2-(meta-Chlorphenyl)-, 2-(para-Chlorphenyl)ethyl); gegebenenfalls substituiertes Styryl (z.B. Styryl; 2'-Chlor-, 3'-Chlor-, 4'-Chlor-, 2,',4'-Dichlor-, 2'-Fluor-, 2'-Methyl-, 4'-Methyl-, 4'-t-Butyl-, 2'-Methoxy-, 4'-Methoxy-, 4'-Phenoxy-styrol); Phenyl-C₃-C₆-alkyl (z.B. 3-Phenylpropyl, 2-Methyl-3-Phenylpropyl, 4-Phenylbutyl, 5-Phenylpentyl, 6-Phenylhexyl, 3-(4'-t-Butylphenyl)-2-Methyl-propyl); Aryloxy, (wenn Y = O, S, Oxycarbonyl, Oxyalkoxy, Oxyimino, Thioalkyl, -N==N-, Oxycarbonylalkyl, Oxyalkyl), wie substituiertes Phenoxy (z.B. 2-Chlor-, 3-Chlor-, 4-Chlor-, 2-Methyl-, 4-Methyl-, 2-Methoxy-, 4-Methoxy-, 2-Trifluormethyl-, 4-Trifluormethyl-phenoxy); Aryloxy-C₁-C₆-alkyl, wie gegebenenfalls substituiertes Phenoxymethyl (z.B. 2-Chlor-, 2-Methyl-, 4-Methyl-, 2-Methoxy-, 4-Methoxy-, 4-t-Butyl-phenoxymethyl); gegebenenfalls substituiertes Phenoxyethyl (z.B. 2-Chlor-, 4-Chlor-, 4-Fluor-, 2-Methyl-, 4-Methyl-, 2-Methoxy-, 4-Methoxy-, 4-t-Butyl-phenoxyethyl), gegebenenfalls substituiertes C₃-C₆-Phenoxyalkyl (z.B. 3-Phenoxypropyl, 3-(ortho-Chlorphenoxy)propyl, 3-(para-Chlor-phenoxy)propyl, 4-Phenoxybutyl, 4-(ortho-Chlorphenoxy)-butyl, 4-(para-Chlorphenoxybutyl), 5-Phenoxypentyl, 5-ortho-Chlorphenoxy)pentyl, 5-(para-Chlorphenoxy)pentyl, 6-Phenoxyhexyl); Phenoxyethoxy (wenn Y = O, S, Oxycarbonyl, Oxyalkoxy, Oxyimino, Thioalkyl, -N==N-, Oxycarbonylalkyl, Oxyalkyl), Methoxycarbonyl, t-Butoxycarbonyl; gegebenenfalls substituiertes Heteroaryl (z.B. Furyl, 2-Furyl, 3-Furyl, 5-Nitro-2- und 3-Furyl, 5-Chlor-2-und 3-Furyl, Benzfuran-2- und 3-yl, Thienyl, 2-Thienyl, 3-Thienyl, 5-Nitro-2- und 3-thienyl, 5-Chlor-2- und 3-thienyl, Benzothien-2- und 3-yl.
N-Methyl-pyrrol-2- und 3-yl, N-Methyl-pyrazol-3-, 4- und 5-yl, N-Methyl-imidazol-2-, 4- und 5-yl, 1-Methyl-1,2,3-triazol-4- und 5-yl, 1-Methyl-1,2,4-triazol-3- und 5-yl, 1-Methyl-tetrazol-5-yl, Isocazol-3-, 4- und 5-yl, Benzisoxazol-3-yl, Benzoxazol-2-yl, Oxazol-2-, 4- und 5-yl, Thiazol-2-, 4- und 5-yl, Benzothiazol-2-yl, Benzisothiazol-3-yl, Iso-thiazol-3-, 4- und 5-yl, 1,2,3-Thiadiazol-4-yl, 1,2,4-Thiadiazol-5-yl, 1,3-4-Thiadiazol-3-yl, 1,3-Thiazolo-[4,5-b]-pyridin-2-yl; substituiertes Hetarylalkenyl (z.B. 2-(2'- und 3'-Furyl)-, 2-(5'-Nitro-2'- und 3'-furyl)-, 2-(2'- und 3'-Thienyl)-, 2-(5'-Nitro-2'- und 3'-thienyl)-ethenyl).
- R: C₃-C₈-Cycloalkyl, gegebenenfalls substituiertes Phenyl, gegebenenfalls substituiertes Hetaryl oder 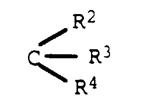 bedeutet,
- R¹: Wasserstoff, C₁-C₆-Alkyl bedeutet,
- R²: Wasserstoff, C₁-C₈-Alkyl, C₃-C₈-Cycloalkyl oder gegebenenfalls durch C₁-C₄-Alkoxy oder Halogen substituiertes Phenyl bedeutet,
- R³: Wasserstoff bedeutet,
- R⁴: Wasserstoff, C₁-C₈-Alkyl, C₃-C₈-Cycloalkyl oder gegebenenfalls durch C₁-C₄-Alkyl, C₁-C₄-Alkoxy oder Halogen substituiertes Phenyl bedeutet,
- R⁵: C₁-C₈-Alkyl, C₁-C₄-Arylalkyl, C₃-C₆-Cycloalkyl, Hydroxy, C₁-C₄-Alkyloxy, NH₂, C₁-C₄-Alkylamino, C₁-C₄-Dialkylamino bedeutet,
- Y: Methylen, Ethylen, Ethenylen, Ethinylen, Methylenoxy, Ethylenoxy, Oxymethylen, Thiomethylen, Carbonyloxy, Carbonyloxymethylen, Aminocarbonyloxy, Methylenthio, Oxycarbonylmethylen, Oxyethylen, Ethylenthio, Thioethylen, -C==N-O-CH₂-, -O-N==C-, -C==N-O, -N==N-Gruppe oder O oder S bedeutet
- Z: ein geradkettiges oder verzweigtes C₁-C₁₀-Alkyl, C₂-C₁₀-Alkenyl, C₂-C₁₀-Alkinyl, Phenyl, Pyridinyl, Pyridazinyl, Pyrimidinyl, Pyrazinyl, Chinolyl, Isochinolyl, Naphthyridyl, Chinoxalyl, Thienyl, Furyl, Pyrrolyl, Oxazolyl, Isooxazolyl, Pyrazolyl, Imidazolyl, Thiazolyl, Isothiazolyl, Benzothiazolyl, Benzoisothiazolyl, Benzoxazolyl, Benzoisoxazolyl, Benzopyrrazolyl, Benzoimidazolyl, Indolyl, Isoindolyl, Benzothiophenyl, Isobenzothiophenyl, Benzofuranyl, Isobenzofuranyl, oder nur, wenn Y nicht Oxymethylen, Oxyethylen, Oxycarbonyl, Oxycarbonylmethylen, Thiomethylen, Thioethylen, -O-N==C-, -N==N-, O, S ist, ein geradkettiges oder verzweigtes C₁-C₁₀-Alkyloxy, C₂-C₁₀-Alkenyloxy, Phenyloxy, Pyridinyloxy, Pyridazinyloxy, Pyrimidinyloxy, Pyrazinyloxy, Chinolyloxy, Isochinolyloxy, Naphthyridyloxy, Chinoxalyloxy, Thenyloxy, Furyloxy, Pyrrolyloxy, Oxazolyloxy, Isoxazolyloxy, Pyrazolyloxy, Imidazolyloxy, Thiazolyloxy, Isothiazolyloxy, Benzothiazolyloxy, Benzoisothiazolyloxy, Benzoxazolyloxy, Benzoisoxazolyloxy, Benzopyrrazolyloxy, Benzoimidazolyloxy, Indolyloxy, Isoindolyloxy, Benzothiophenyloxy, Isobenzothiophenyloxy, Benzofuranyloxy, Isobenzofuranyloxy, oder nur wenn Y nicht Oxymethylen, Oxyethylen, Oxccarbonyl, Oxycarbonylmethylen, -O-N==N-, O ist, ein geradkettiges oder verzweigtes C₁-C₁₀-Alkylthio, C₂-C₁₀-Alkenylthio, Phenylthio, Pyridinylthio, Pyridazinylthio, Pyrimidinylthio, Pyrazinylthio, Chinolylthio, Isochinolylthio, Naphthyridylthio, Chinoxalylthio, Thienylthio, Furylthio, Pyrrolylthio, Oxazolylthio, Isoxazylthio, Pyrazolylthio, Imidazolylthio, Thiazolylthio, Isothiazolylthio, Benzothiazolylthio, Benzoisothiazolylthio, thiazolylthio, Benzothiazolylthio, Benzoisothiazolylthio, Benzoimidazolylthio, Indolylthio, Isoindolylthio, Benzothiophenylthio, Isobenzothiophenylthio, Benzofuranylthio, Isobenzofuranylthio bedeutet, wobei die für Z genannten Reste gegebenenfalls substituiert sind mit Halogen, Cyano, geradkettigem oder verzweigtem C₁-C₄-Alkyl, C₂-C₄-Alkenyl, C₂-C₄-Alkinyl, Hydroxyimino, C₁-C₄-Carboxyalkoxy, C₁-C₄-Alkanoyl, C₁-C₄-Alkoxyalkyl, C₁-C₄-Dialkoxyalkyl, O-Alkyloxyimino, O-Alkenyloxyimino, O-Arylalkyloxyimino, C₁-C₄-Dialkoxy-C₁-C₄-alkyl, Formyl,
- X: Wasserstoff, Halogen, Cyano, geradkettiges oder verzweigtes C₁-C₄-Alkyl, C₁-C₄-Halogenalkyl, C₁-C₄-Alkoxy bedeutet und
- m: eine ganze Zahl zwischen 1 und 4 bedeutet.

Die Verbindungen der Formel Ib, Ic lassen sich herstellen, indem man eine Phenylglyoxylsäuremethylester-O-alkyloxim (EP 253 213) der Formel II 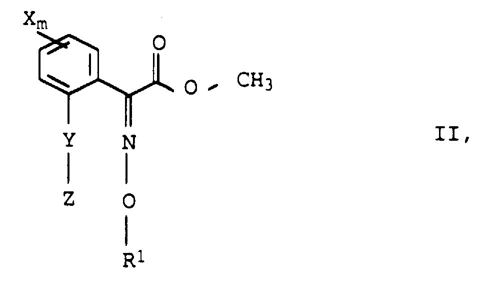 in der R¹, Y, Z, X, m die oben genannte Bedeutung haben, mit einer Verbindung der Formel III 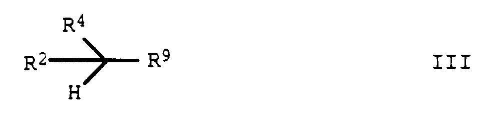 in der R² und R⁴ die oben genannte Bedeutung haben und R⁹ gleich -CN oder -COO-A bedeutet, wobei A die oben genannte Bedeutung hat, unter basischen Bedingungen umsetzt (E.V.P. Tao, G.S. Staten Org.Prep.Proc.Int. Briefs 17 (1985) 235).

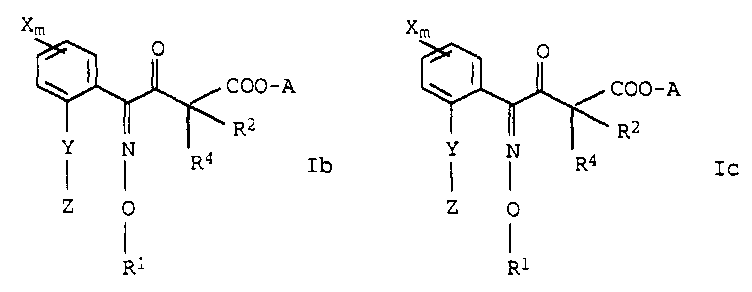

Die Verbindungen Ib lassen sich weiter zu Verbindungen des Typs Ia umsetzen (S. Takei, Y. Kawano, Tetrahedron Lett. 49 (1975) 4389).

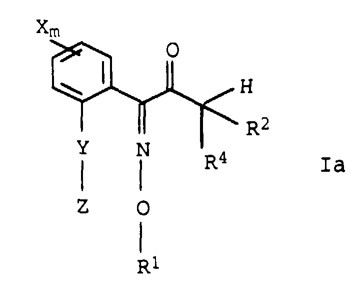

Ausgehend von Verbindungen der Formel II lassen sich die Verbindungen der Formel Id auf einem anderen Weg gewinnen. In bekannter Weise kann man die Verbindungen der Formel IV leicht herstellen (S. Nahm, S.M. Weinreb, Tetrahedron Lett. 39 (1981) 3815).

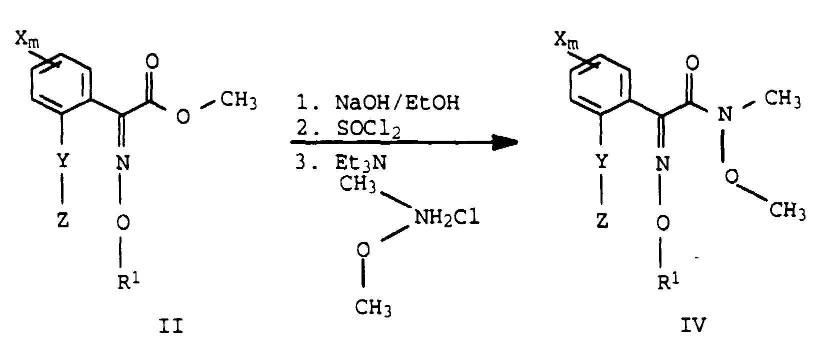

Die weitere Umsetzung der Amide IV mit Organolithiumreagenzien (S. Nahm, S.M. Weinreb, Tetrahedron Ltt. 39 (1981) 3815), liefert die gewünschten α-Oxyiminoketone der Formel Id.

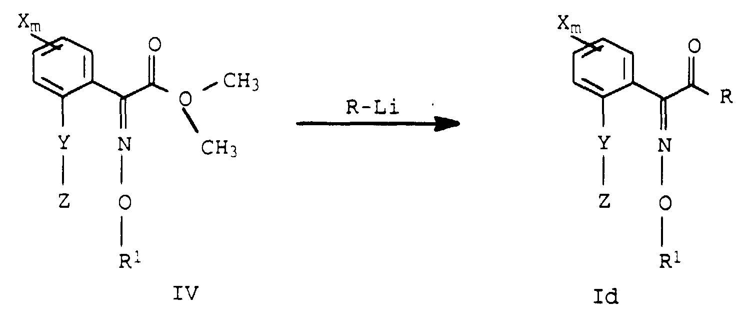

Es ist auch bereits bekannt, daß Verbindungen des Typs Id mit Aminen, Hydroxylaminen und Hydrazinen reagieren und Verbindungen des Typs I liefern (S. 804-805 Advanced Organic Chemistry J. March dritte Auflage).

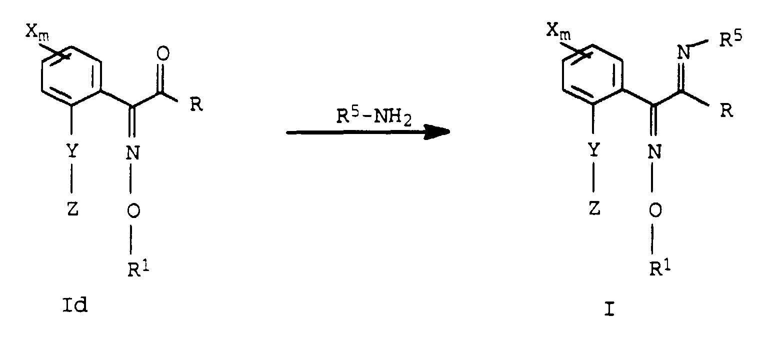

Die Herstellung der neuen Verbindungen und der Zwischenprodukte wird durch die folgenden Beispiele und Vorschriften erläutert:

### Beispiel 1

### 1-Methoxyimino-1-[2'-(2-methylphenoxymethyl)phenyl]-2-Methylimino-Propan (Verbindung Nr. 190)

4 g 1-Methoxyimino-1-[2'-(2-methylphenoxymethyl)phenyl]-Propan-2-on (0,013 mol) wird in 60 ml Ethanol gelöst, 4 g Na₂SO₄ zugegeben, bei -40°C abgekühlt, innerhalb von 20 Minuten 13 g Methylamin eingeleitet, die Kühlung entfernt und das Gemisch 72 h bei Raumtemperatur gerührt. Das Natriumsulfat wird abgesaugt, das Ethanol eingeengt, der Rückstand in MTB-Ether gelöst, die organische Phase dreimal mit Wasser gewaschen, getrocknet und eingeengt. Man erhält 3,2 g (80 %) Verbindung Nr. 675, Fp. 102-104°C.

In entsprechender Weise werden die in der folgenden Tabelle aufgeführten Verbindungen hergestellt.

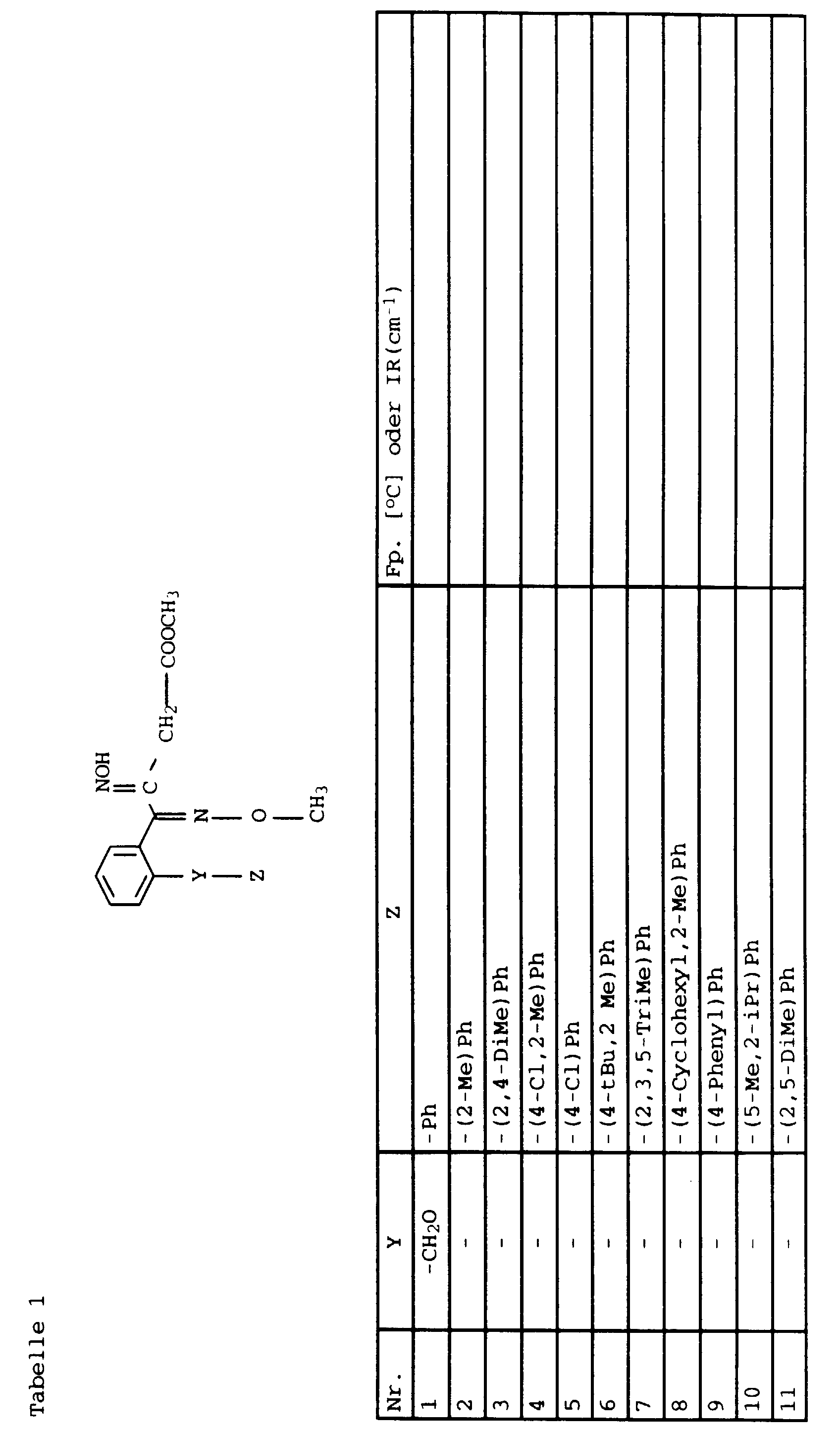

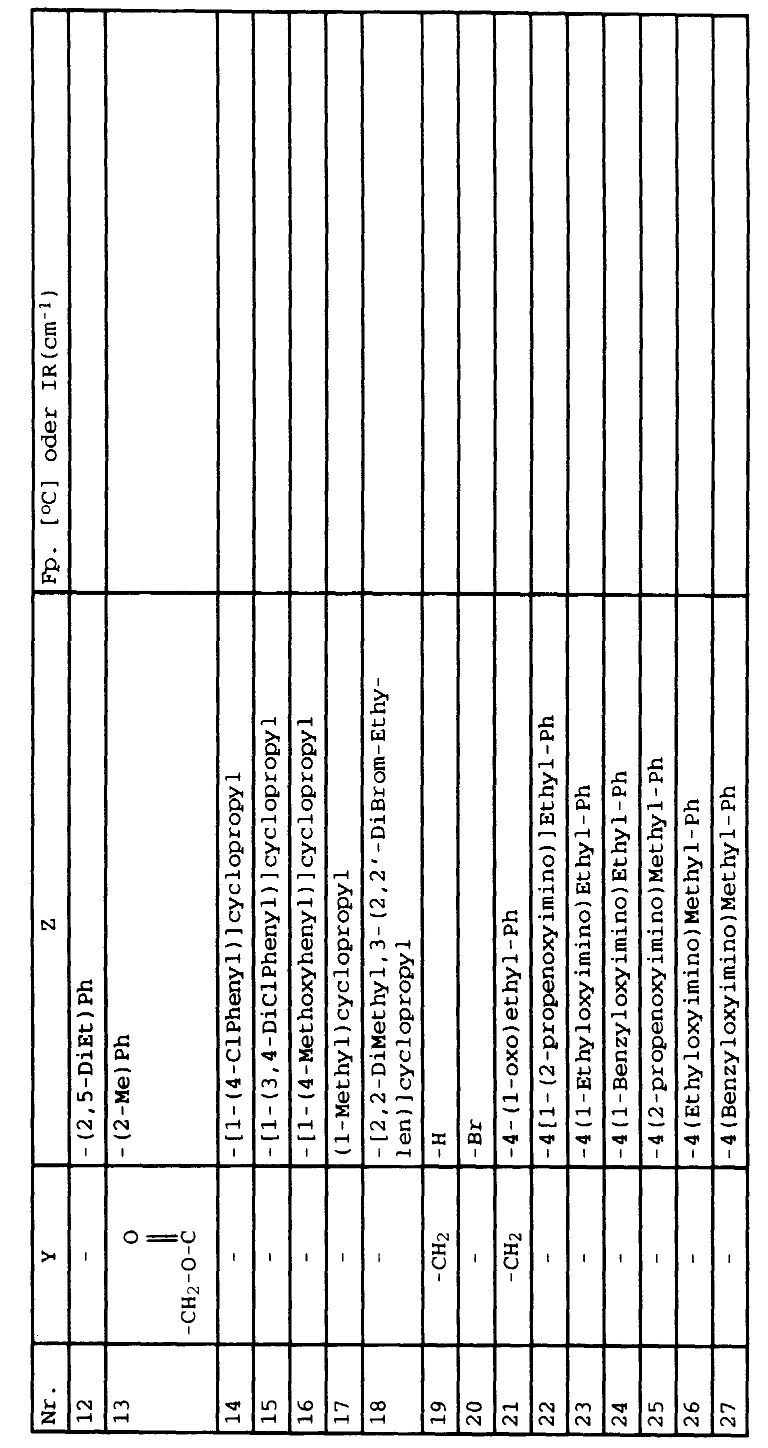

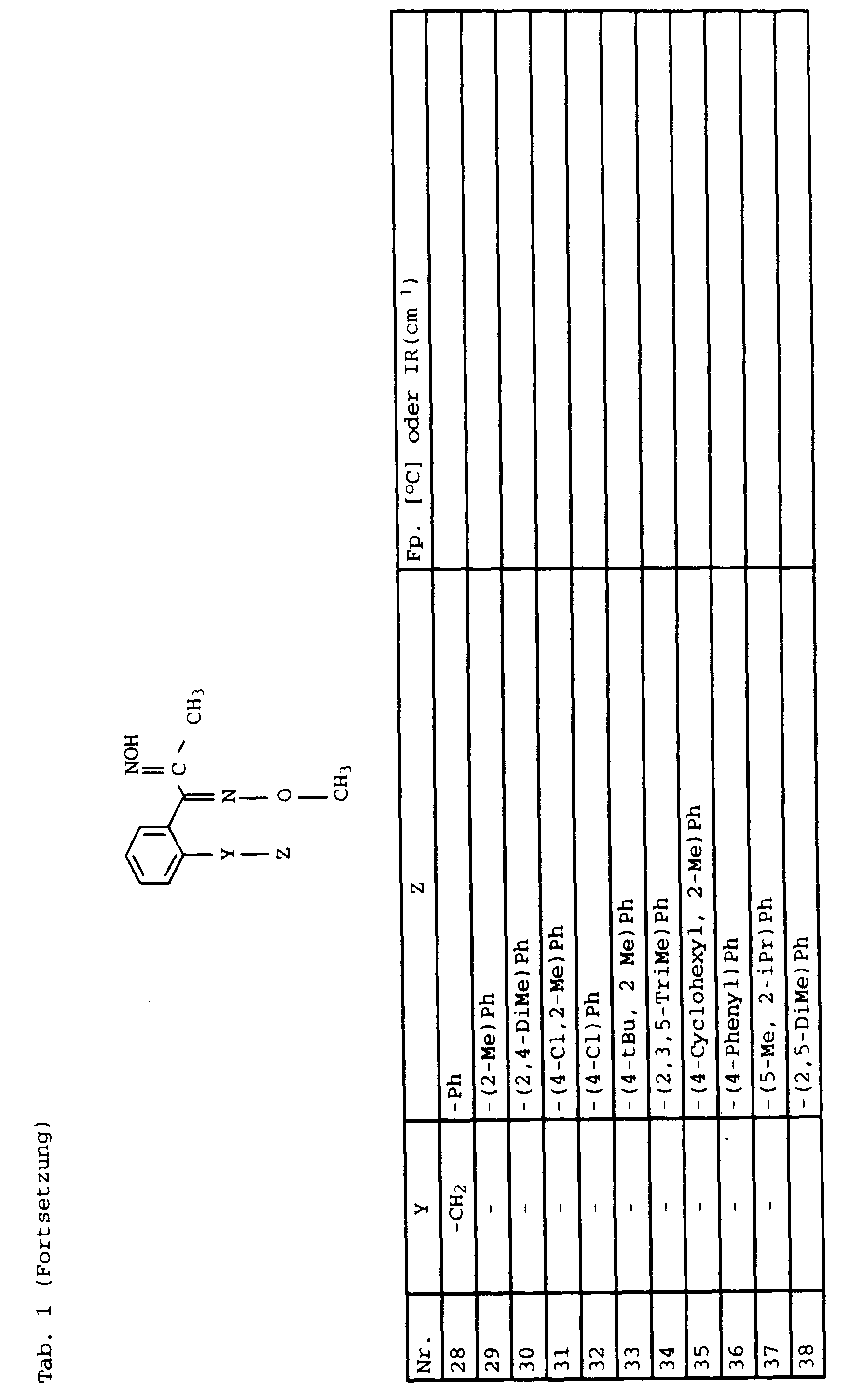

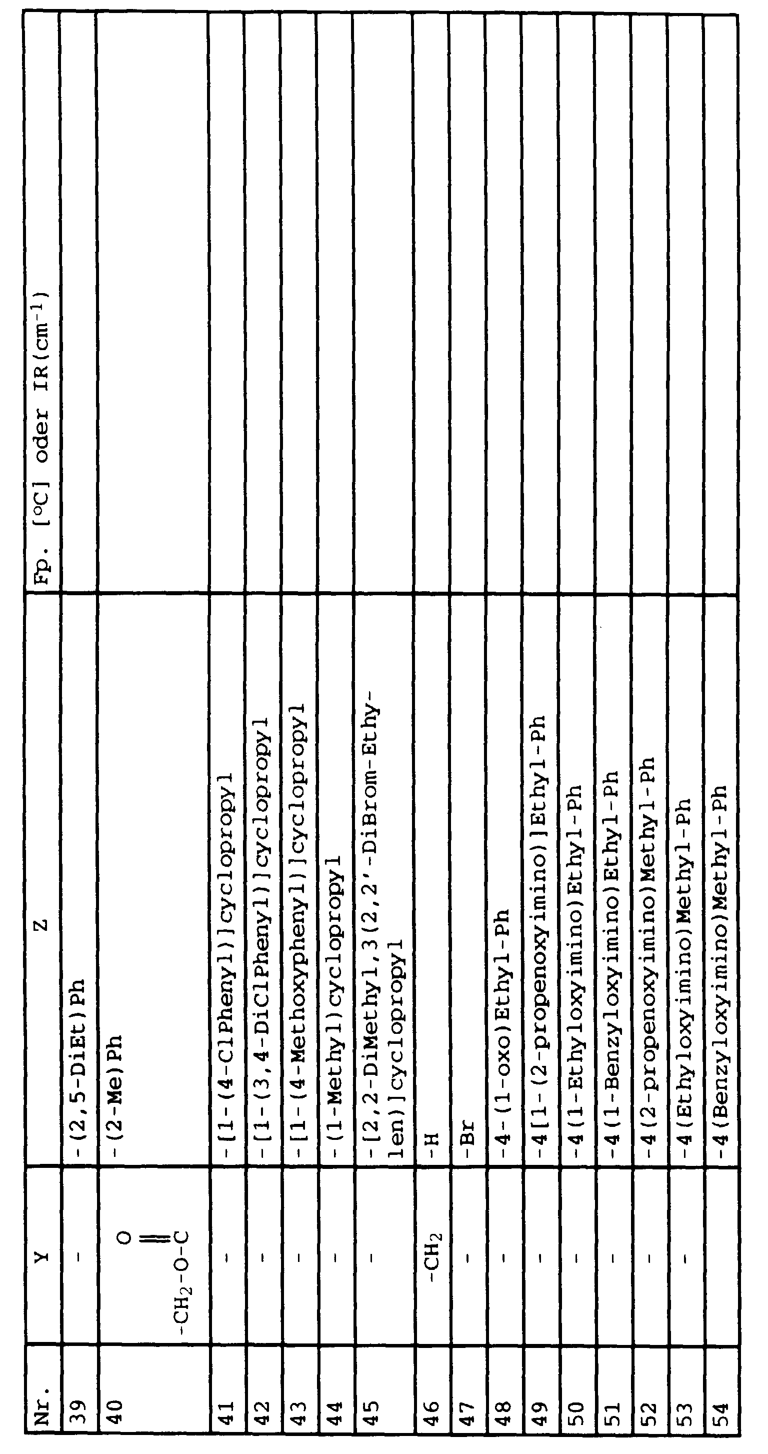

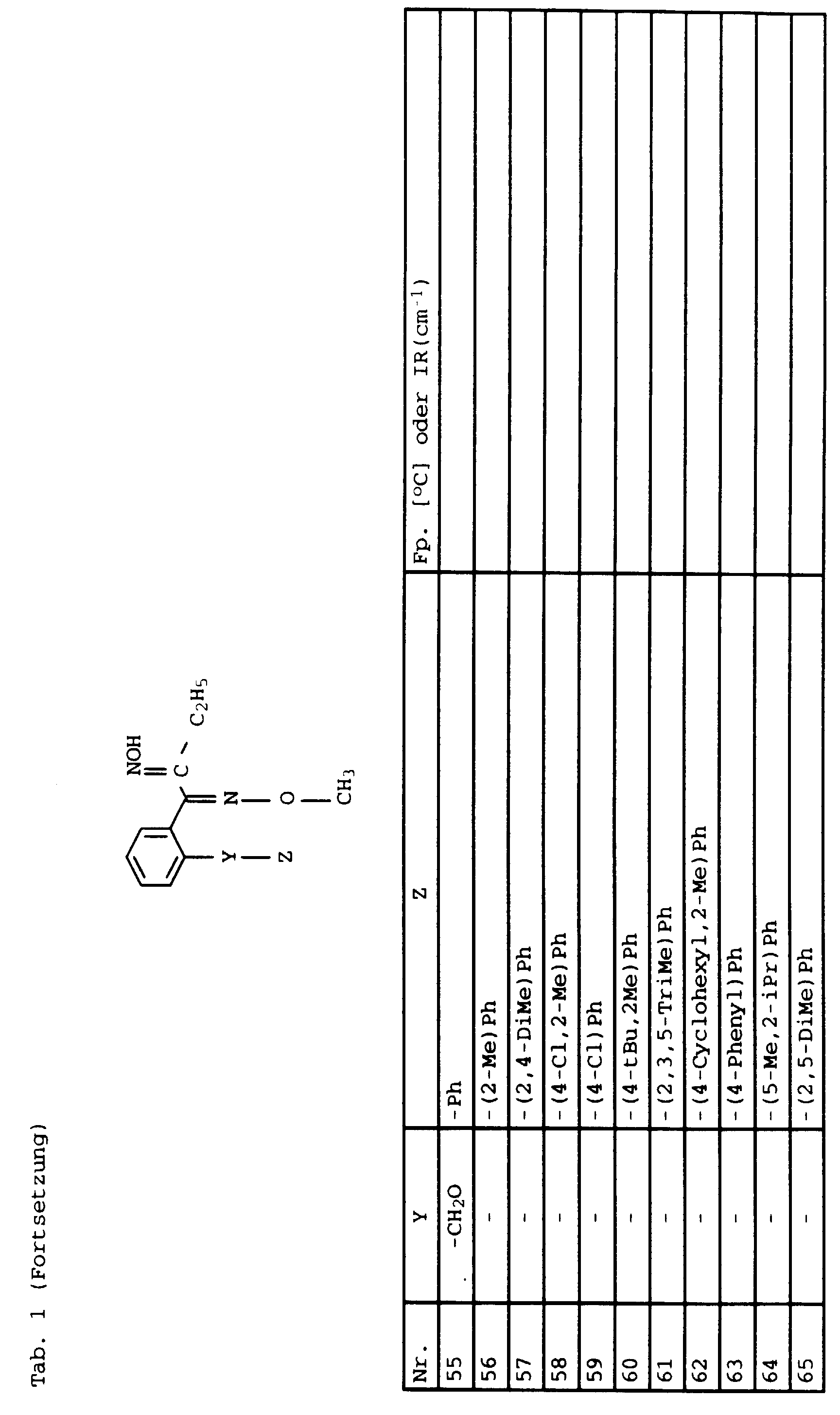

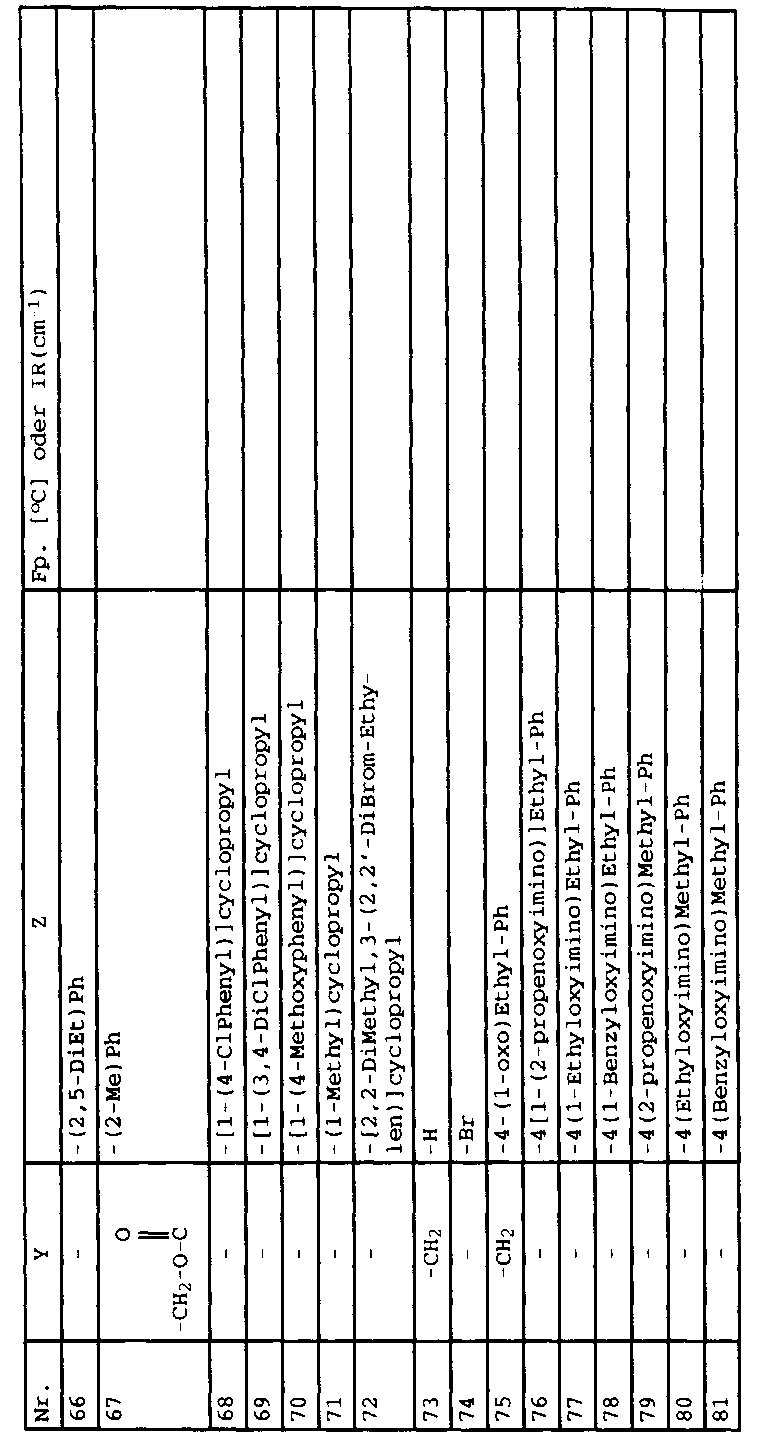

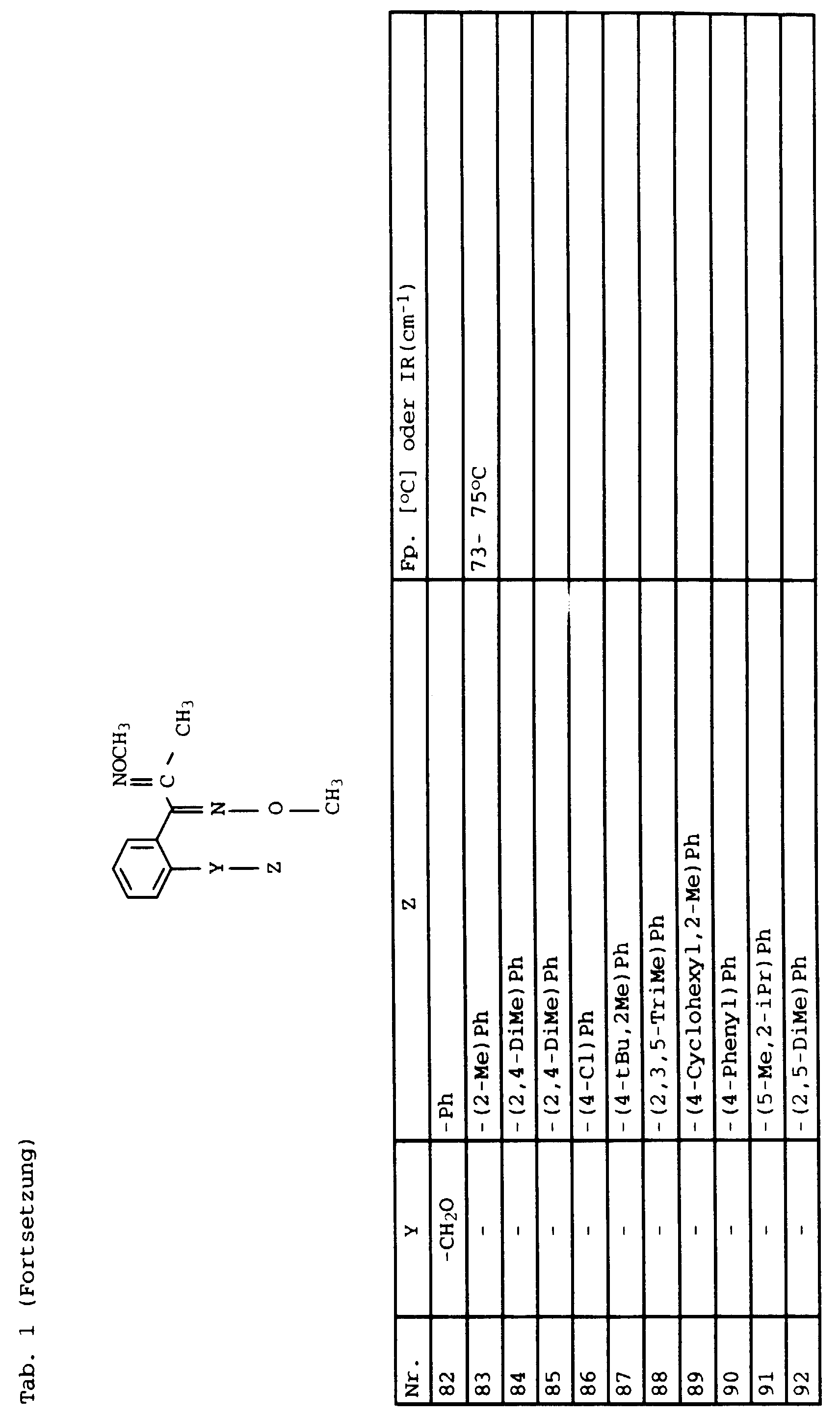

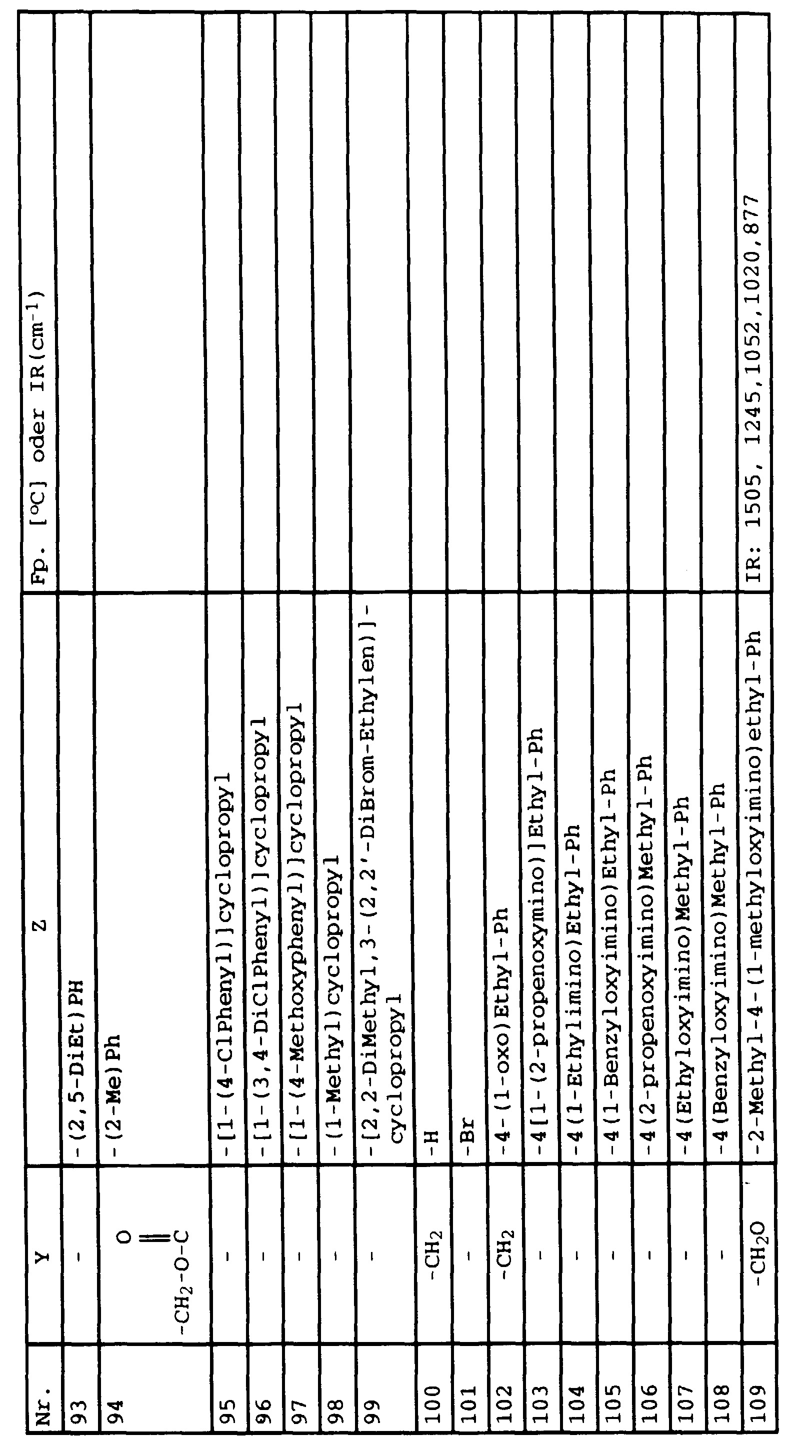

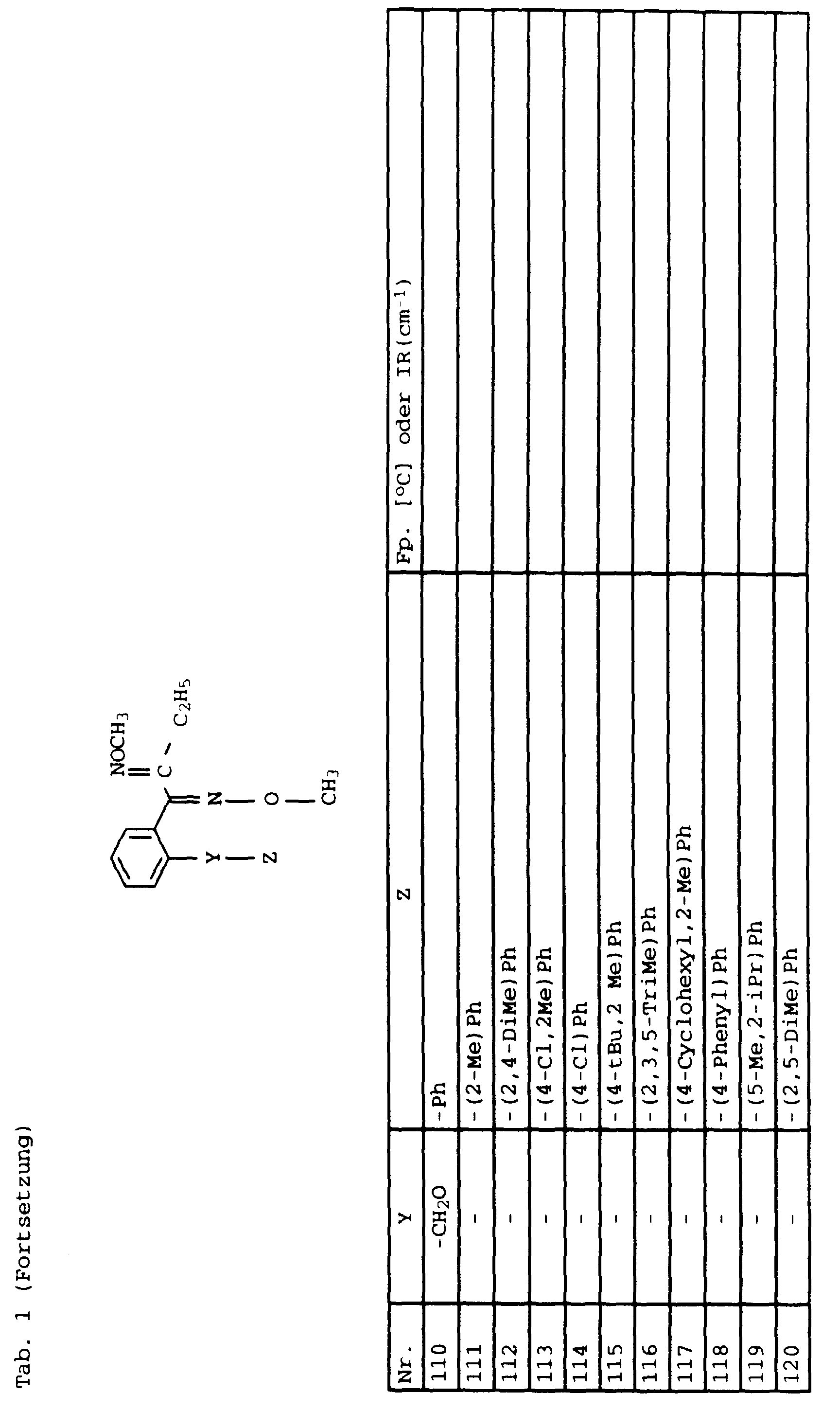

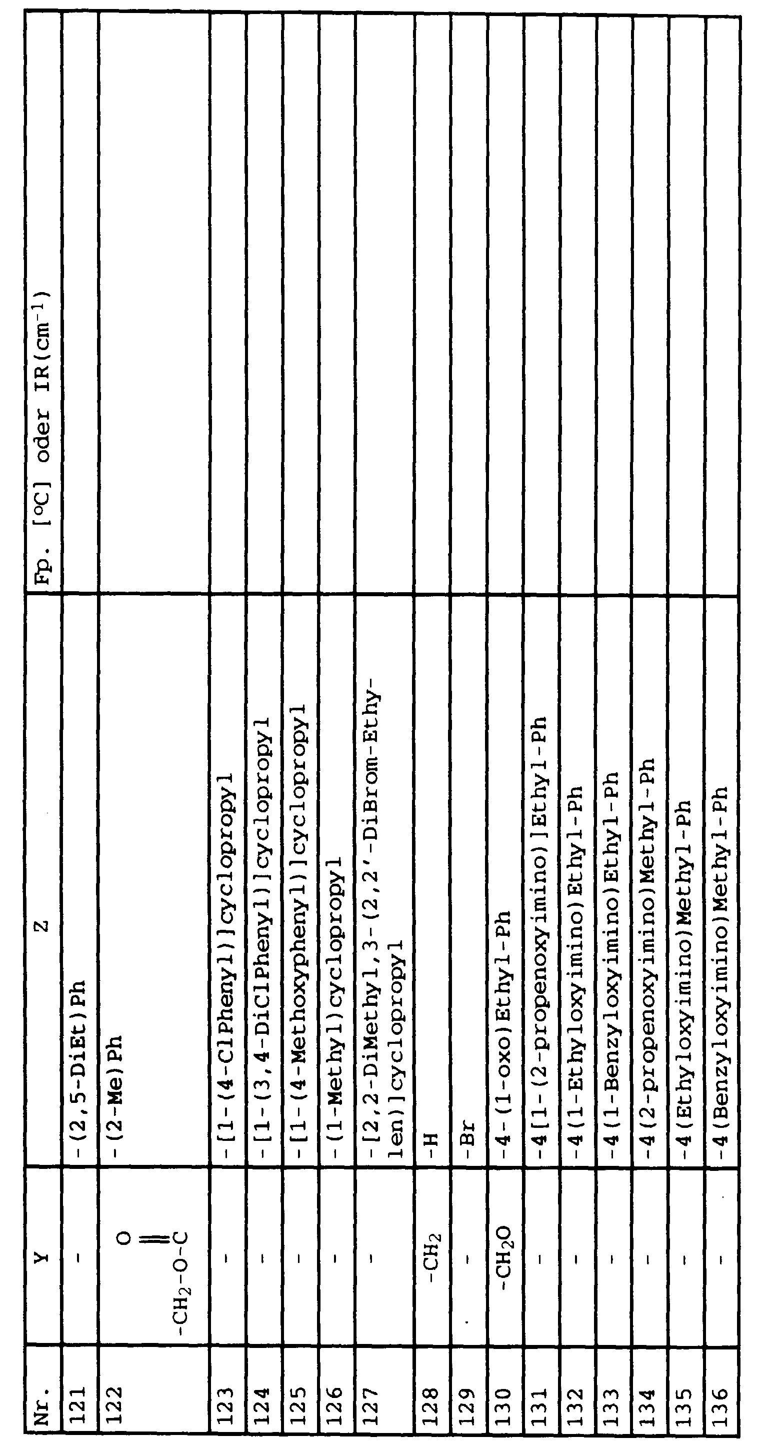

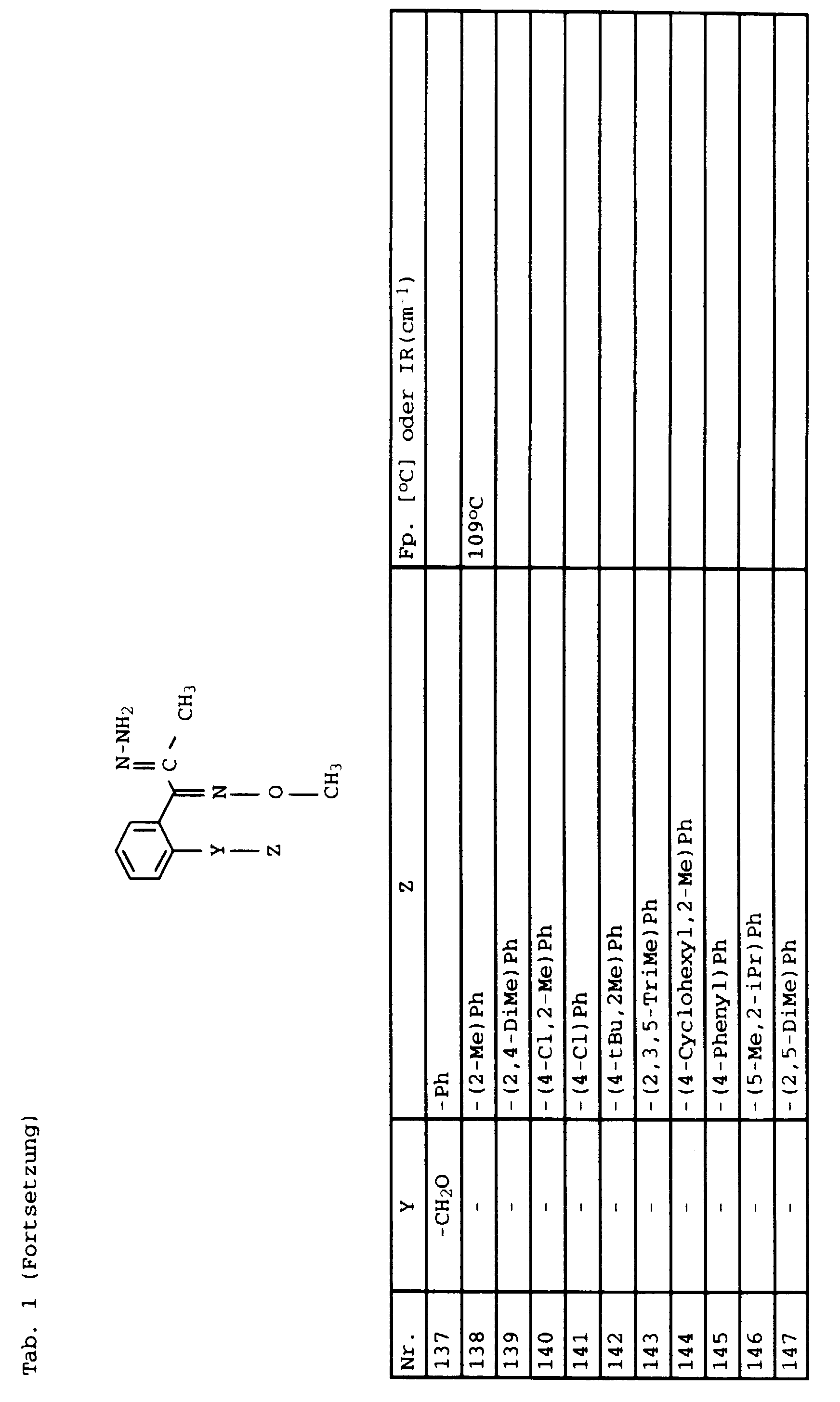

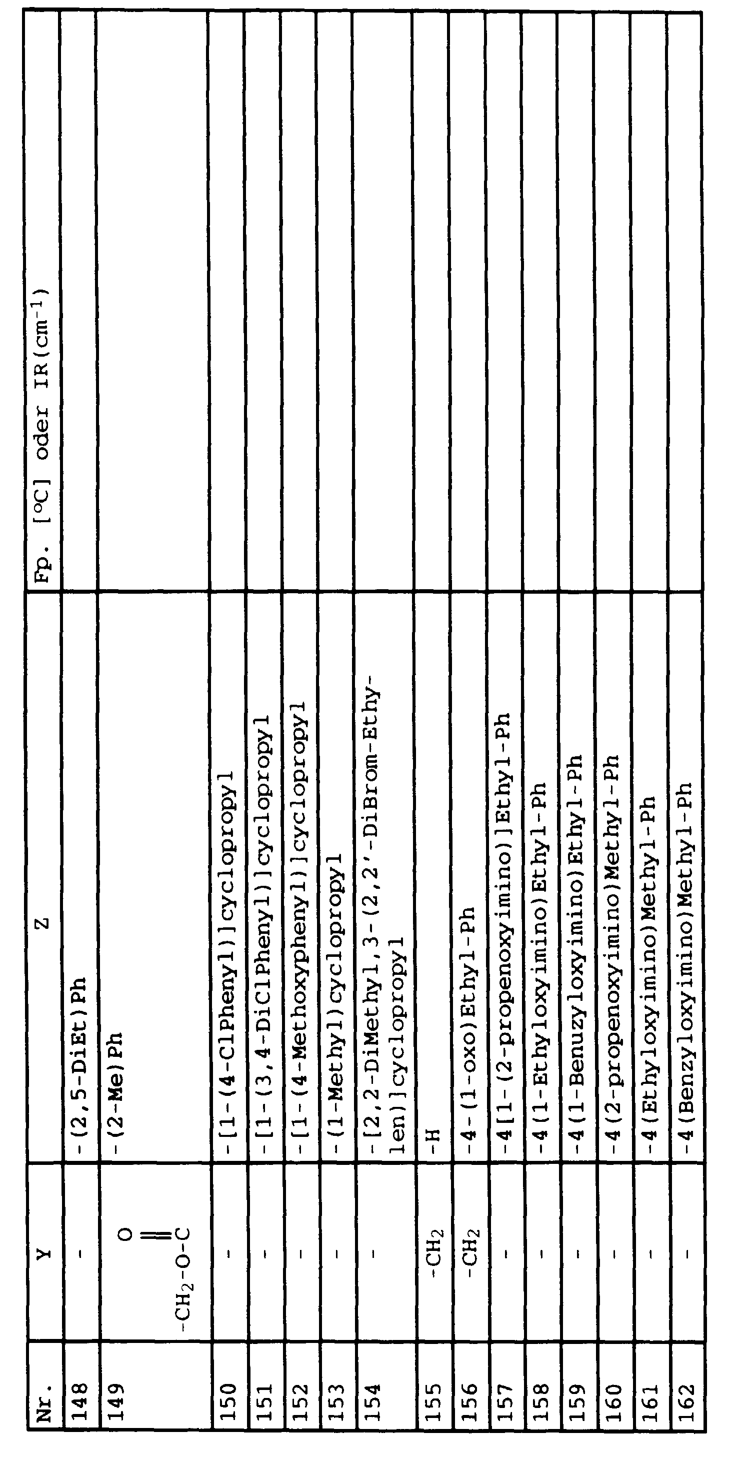

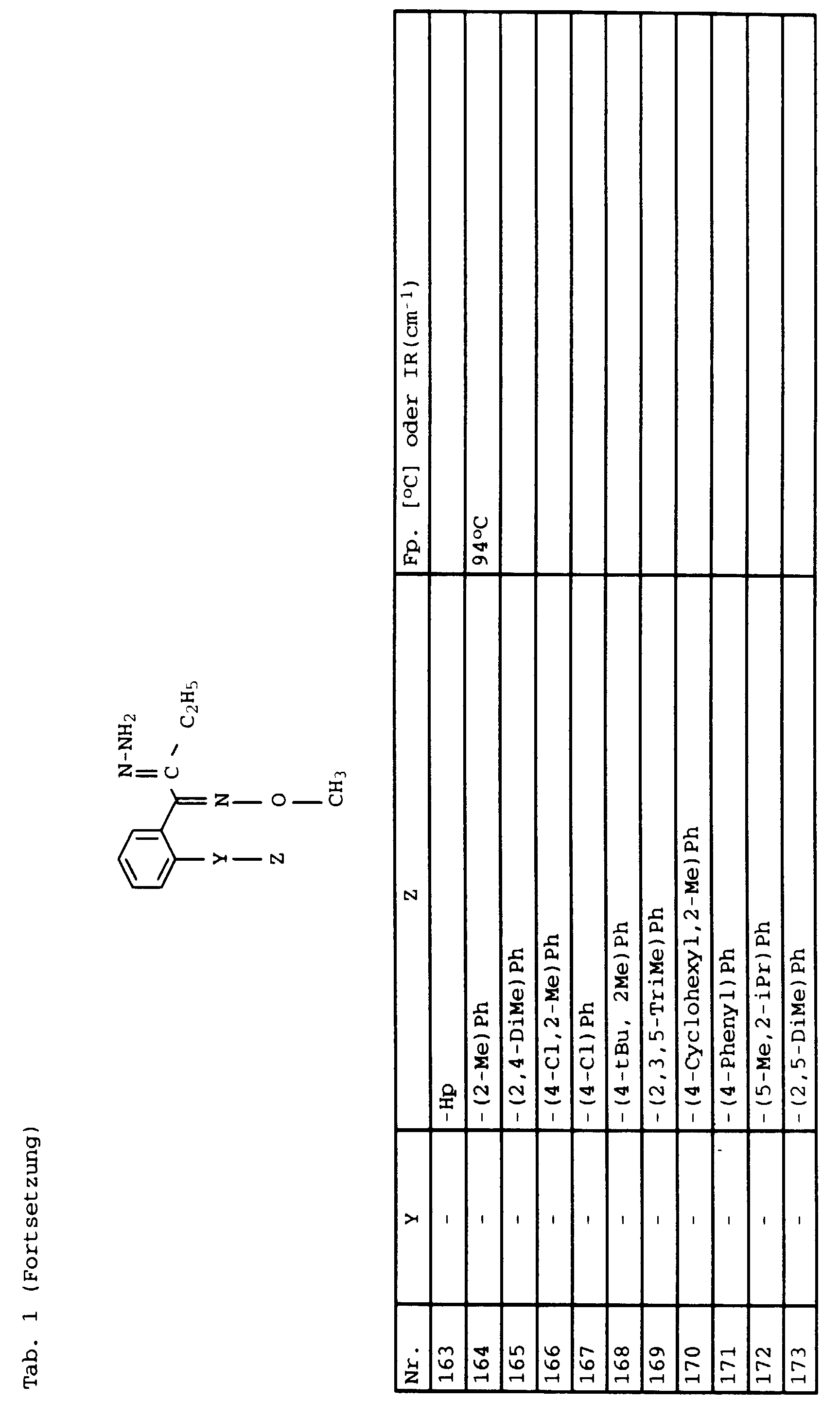

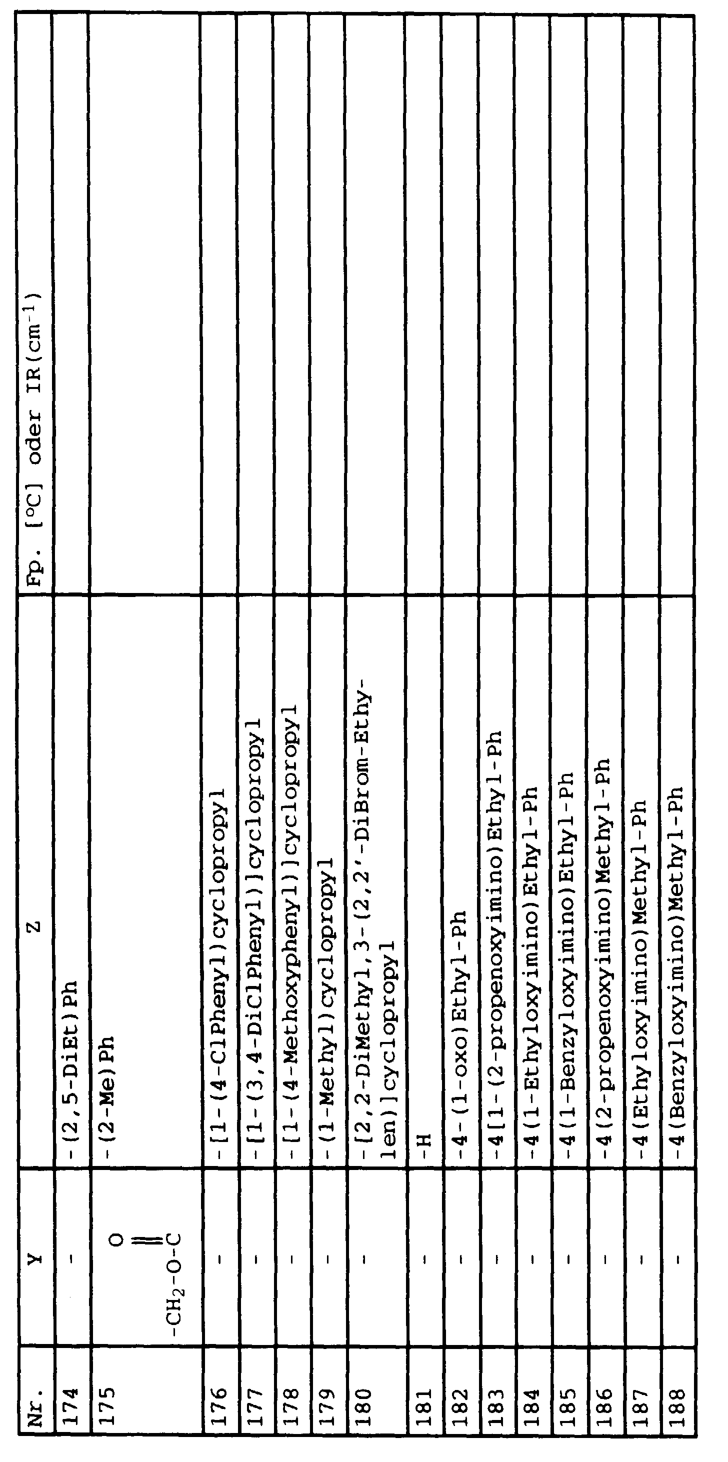

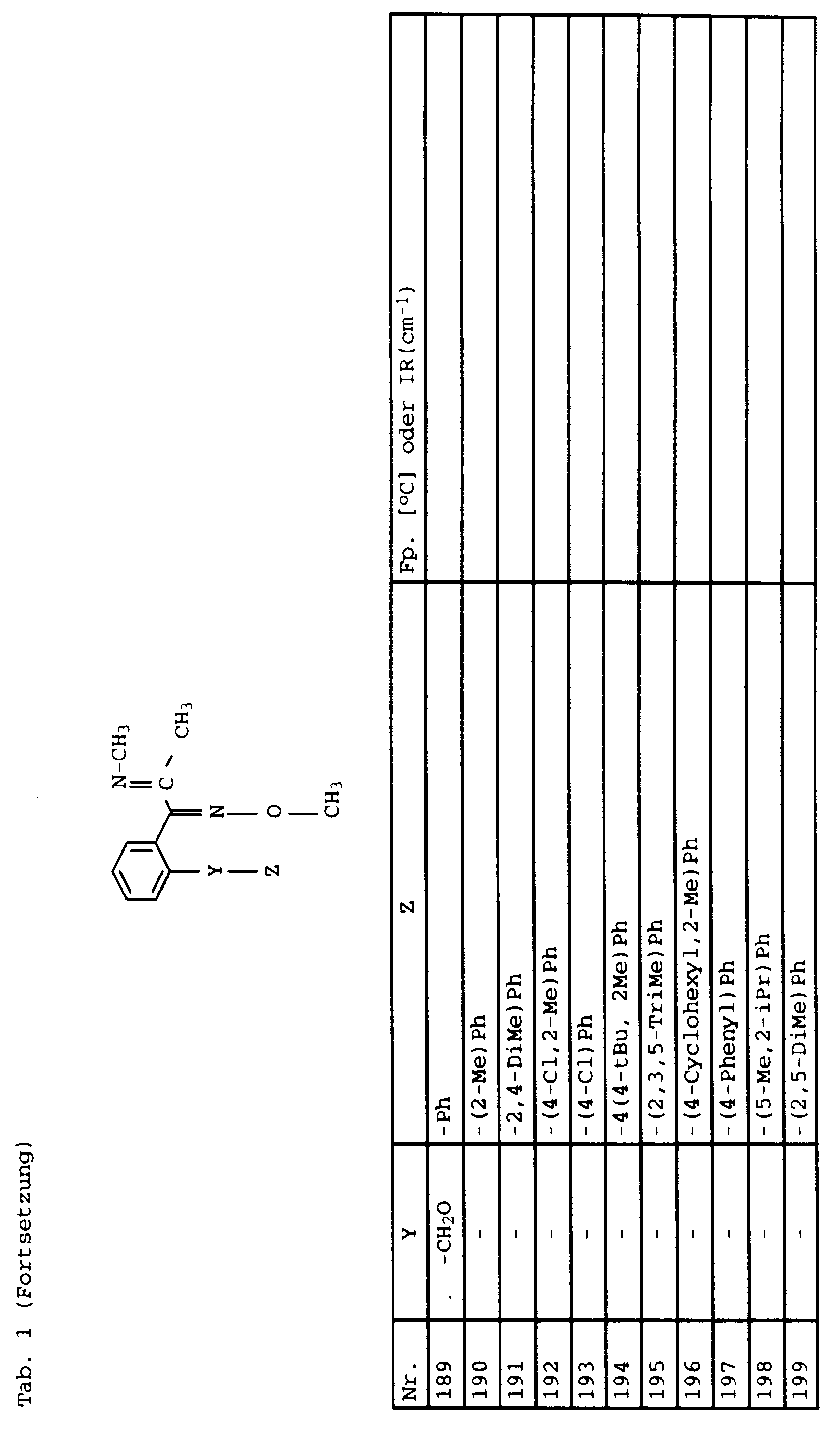

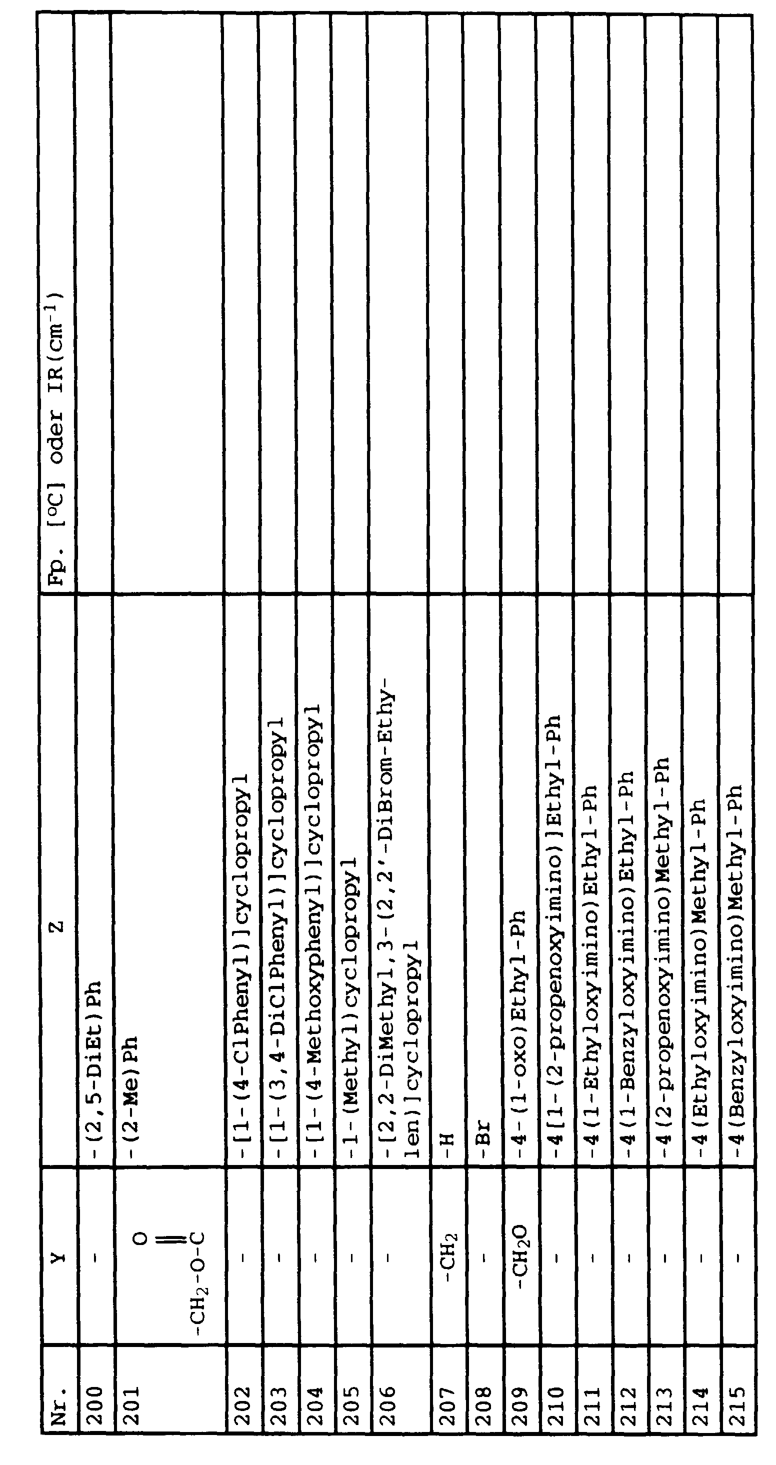

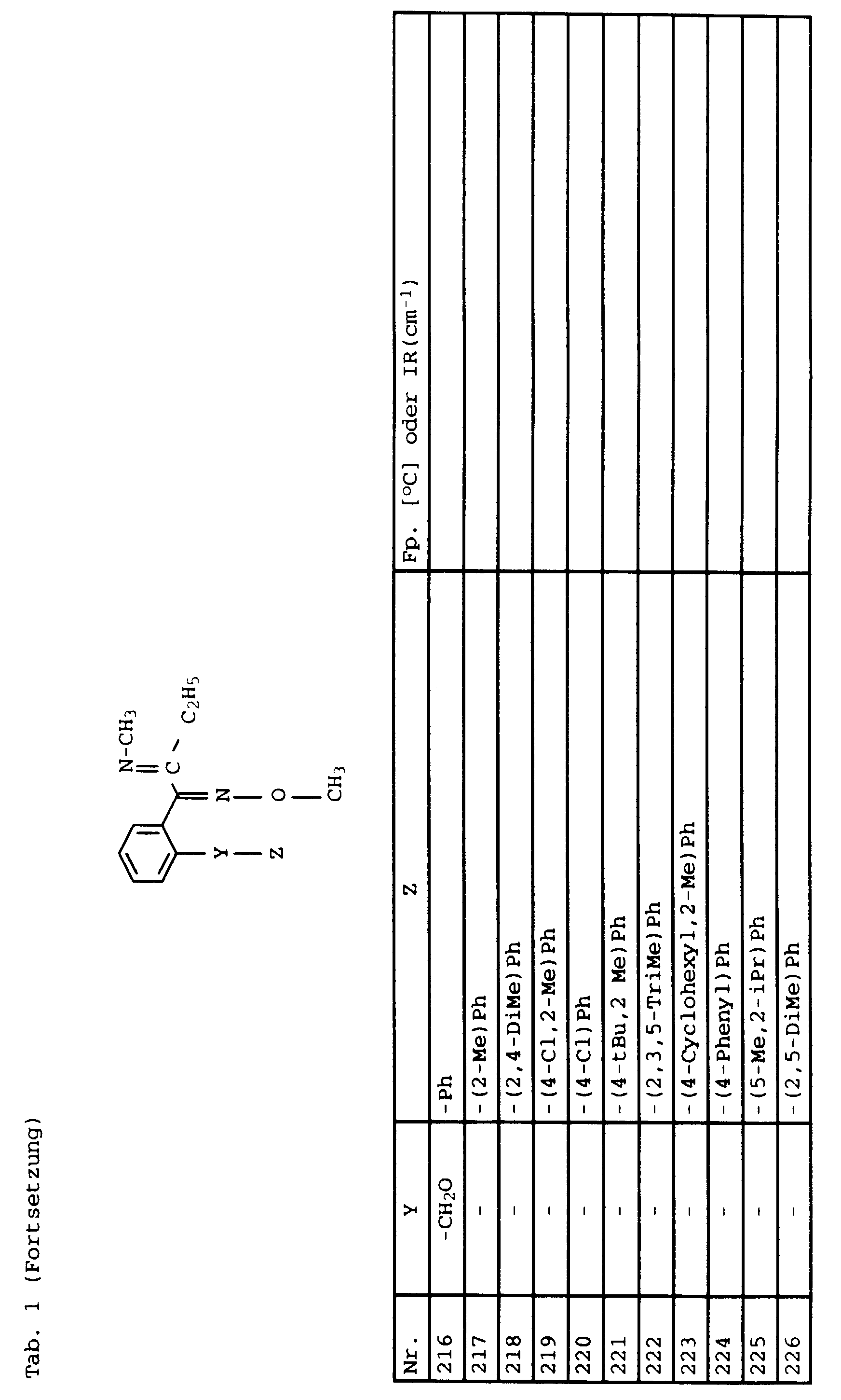

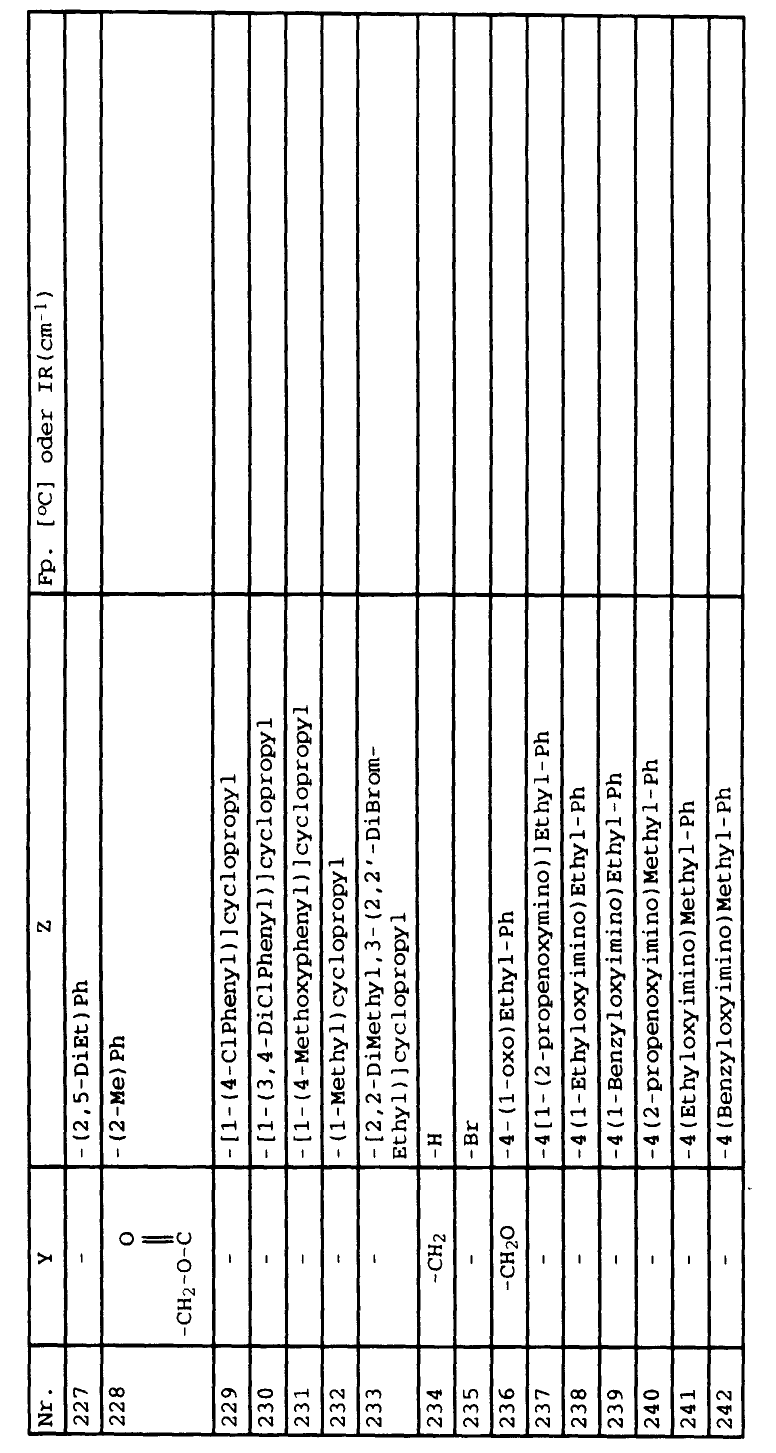

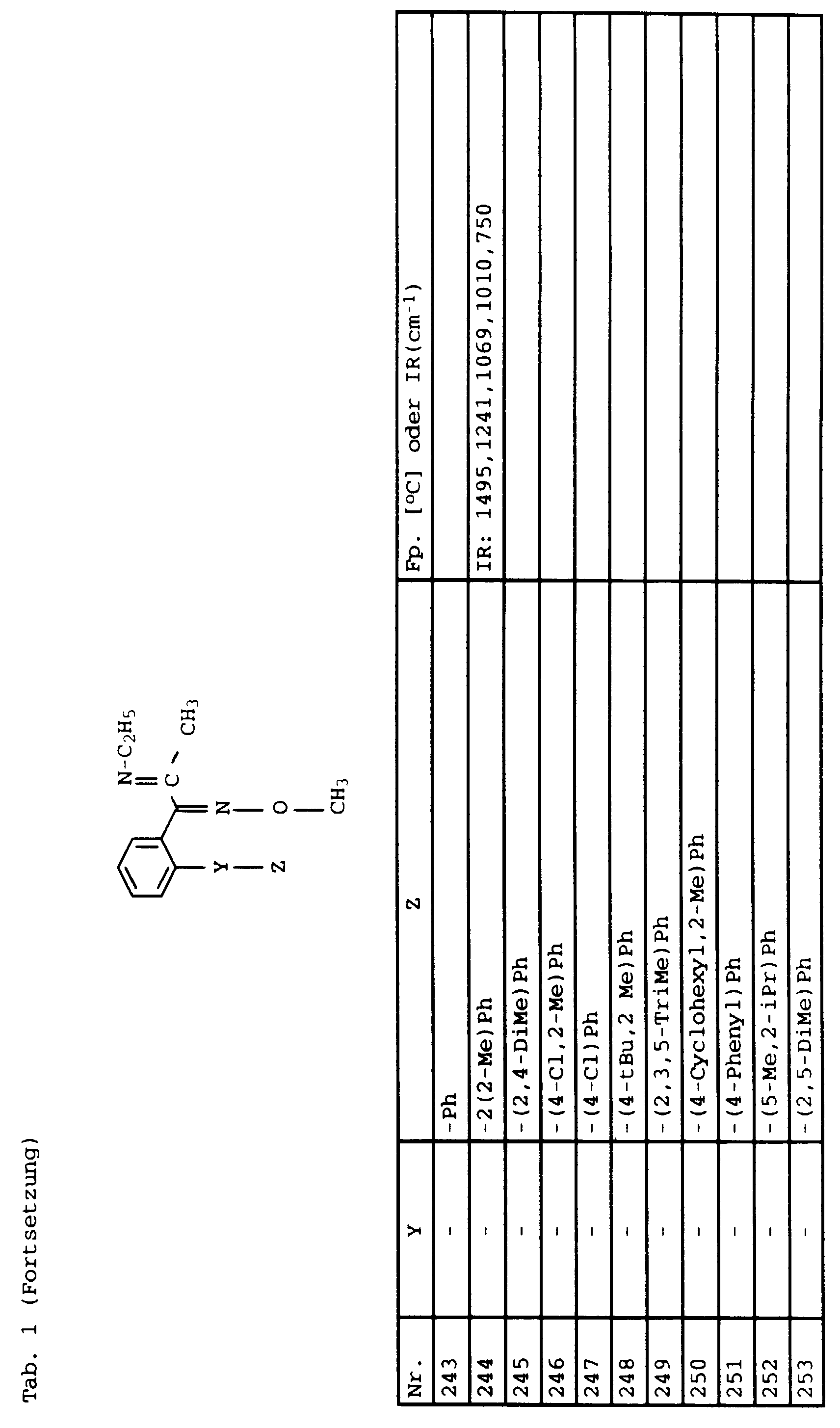

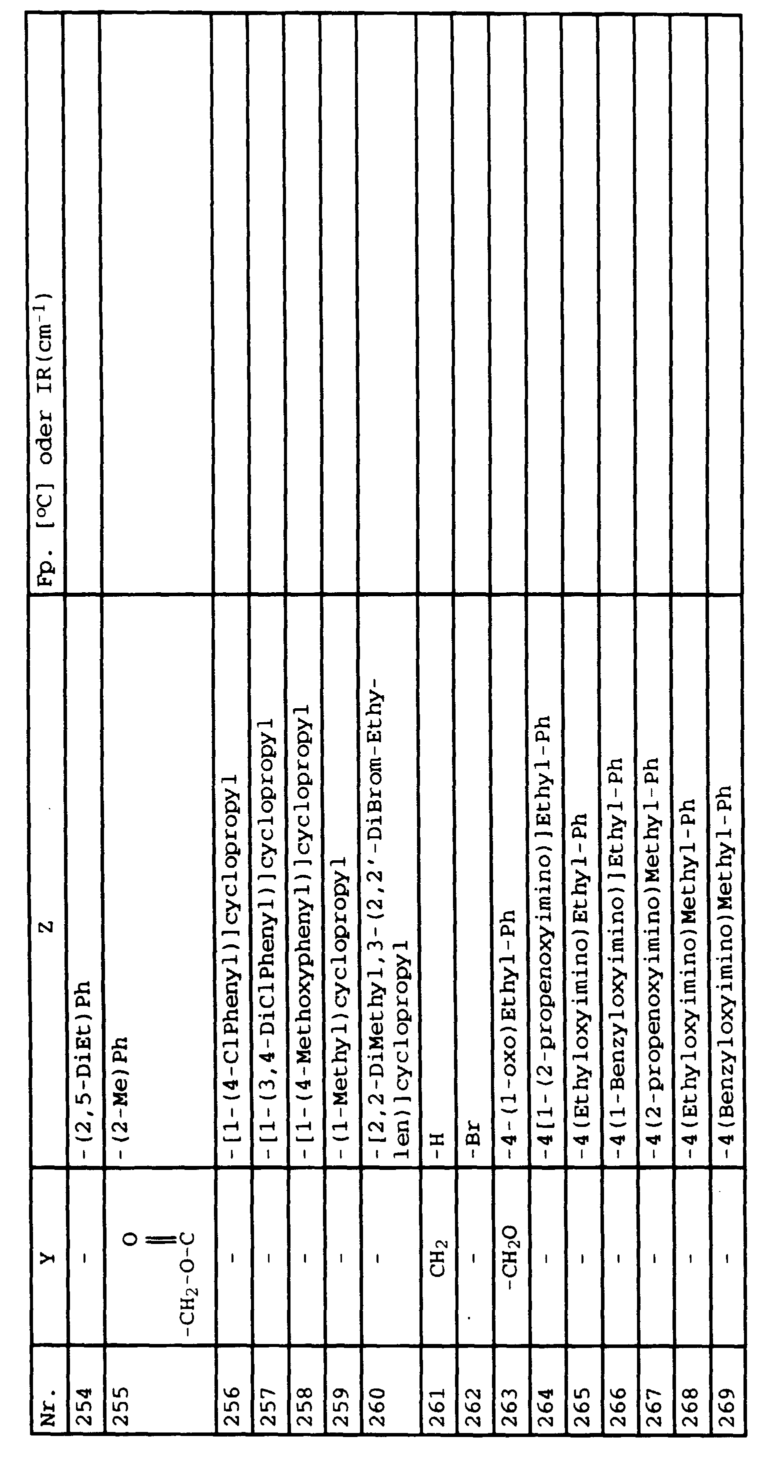

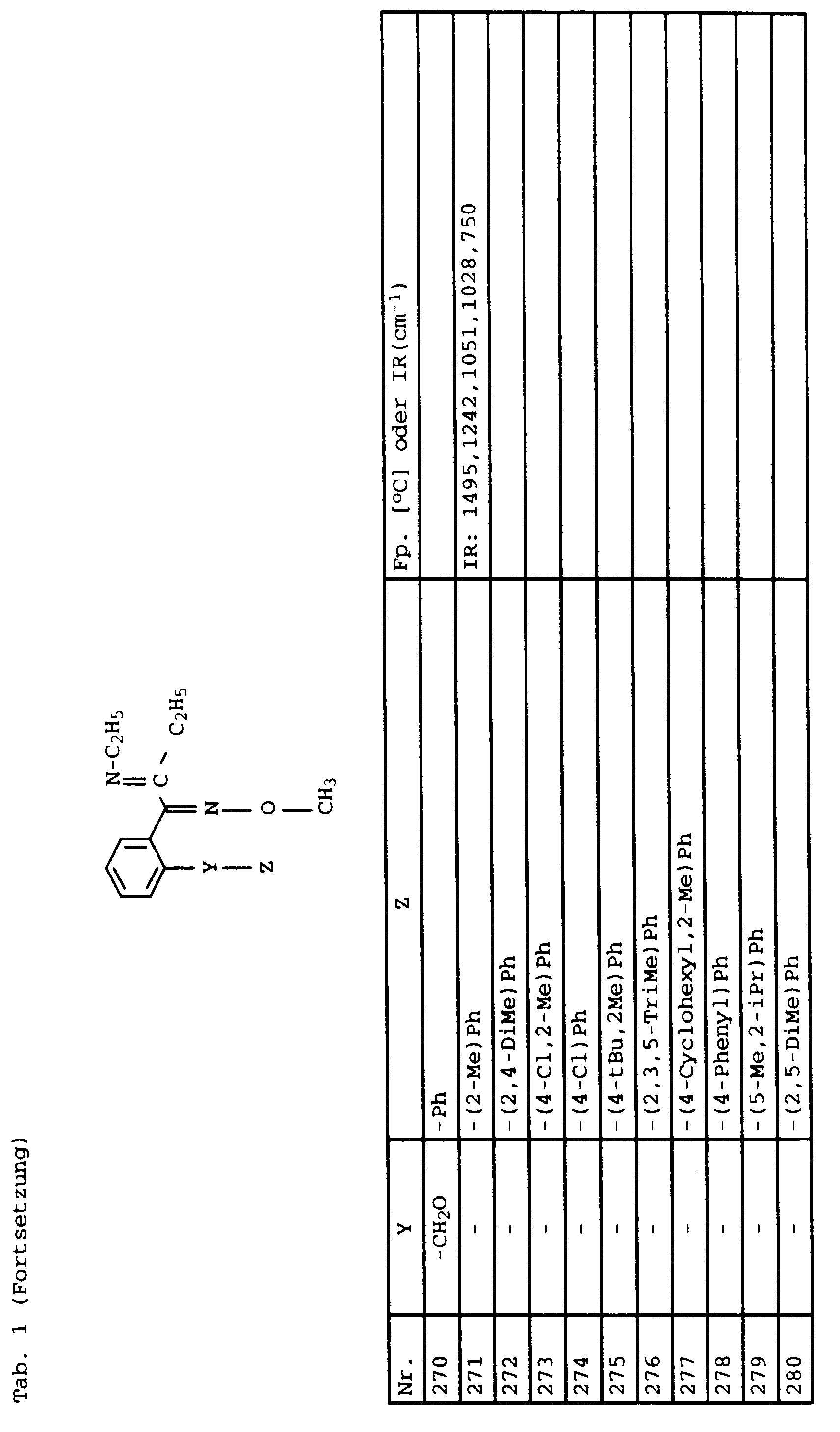

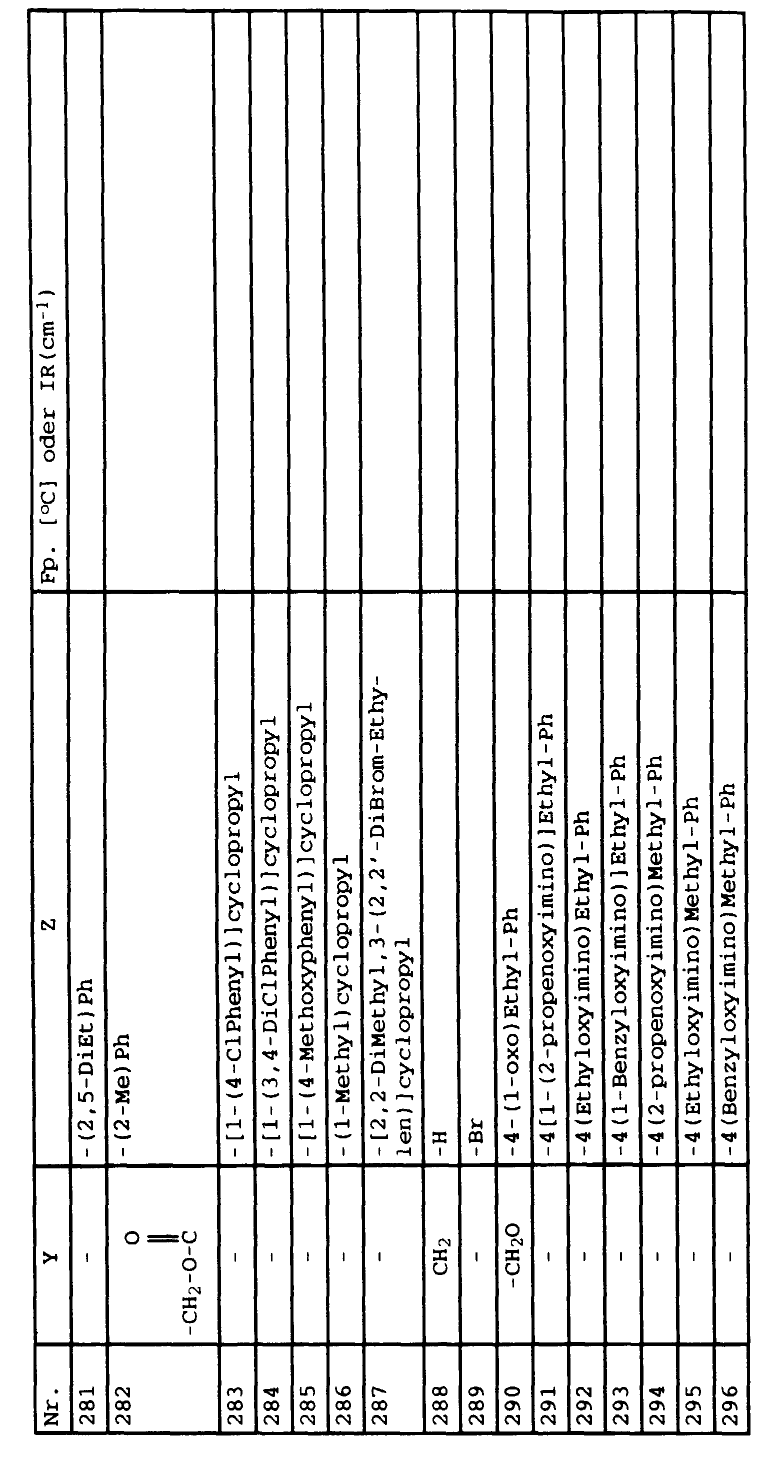

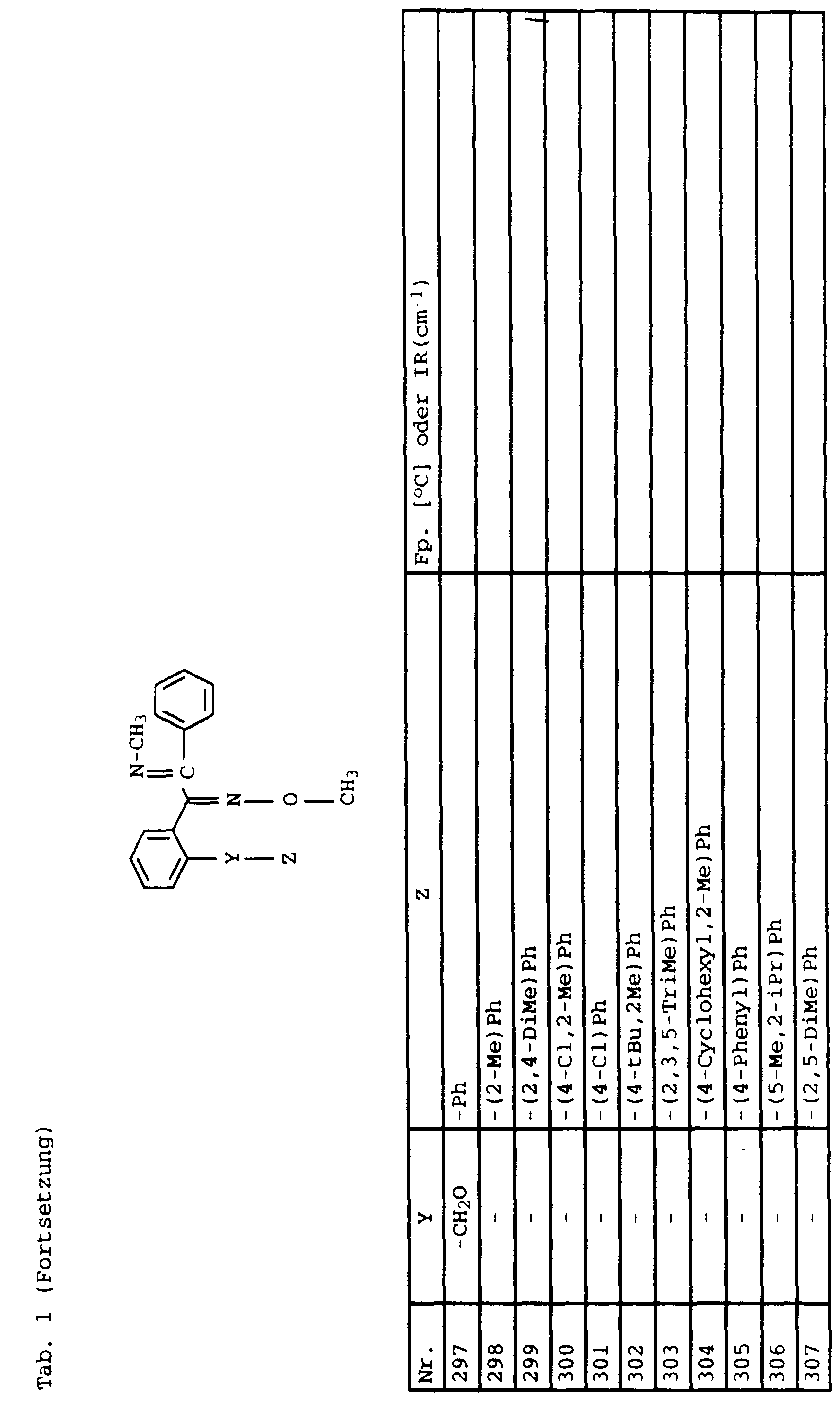

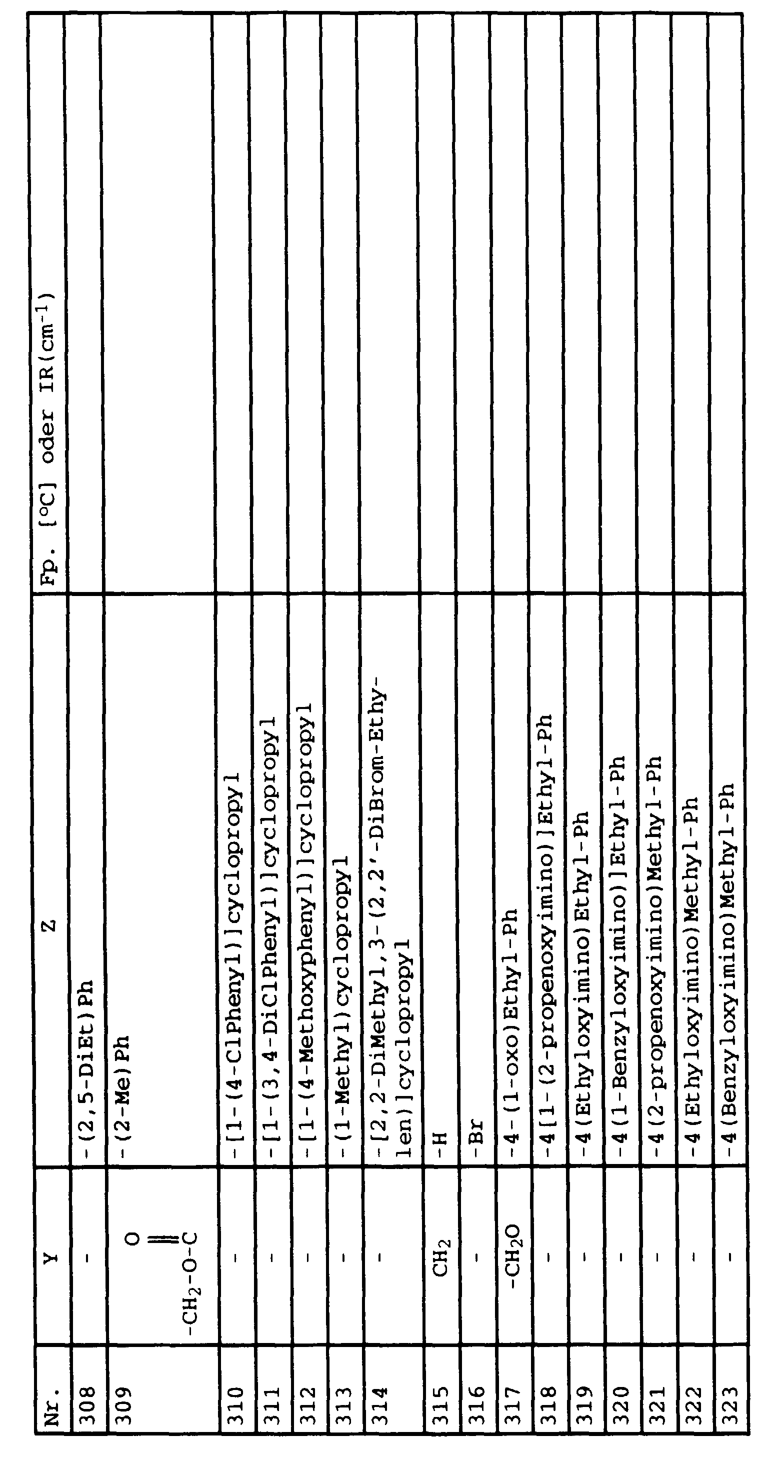

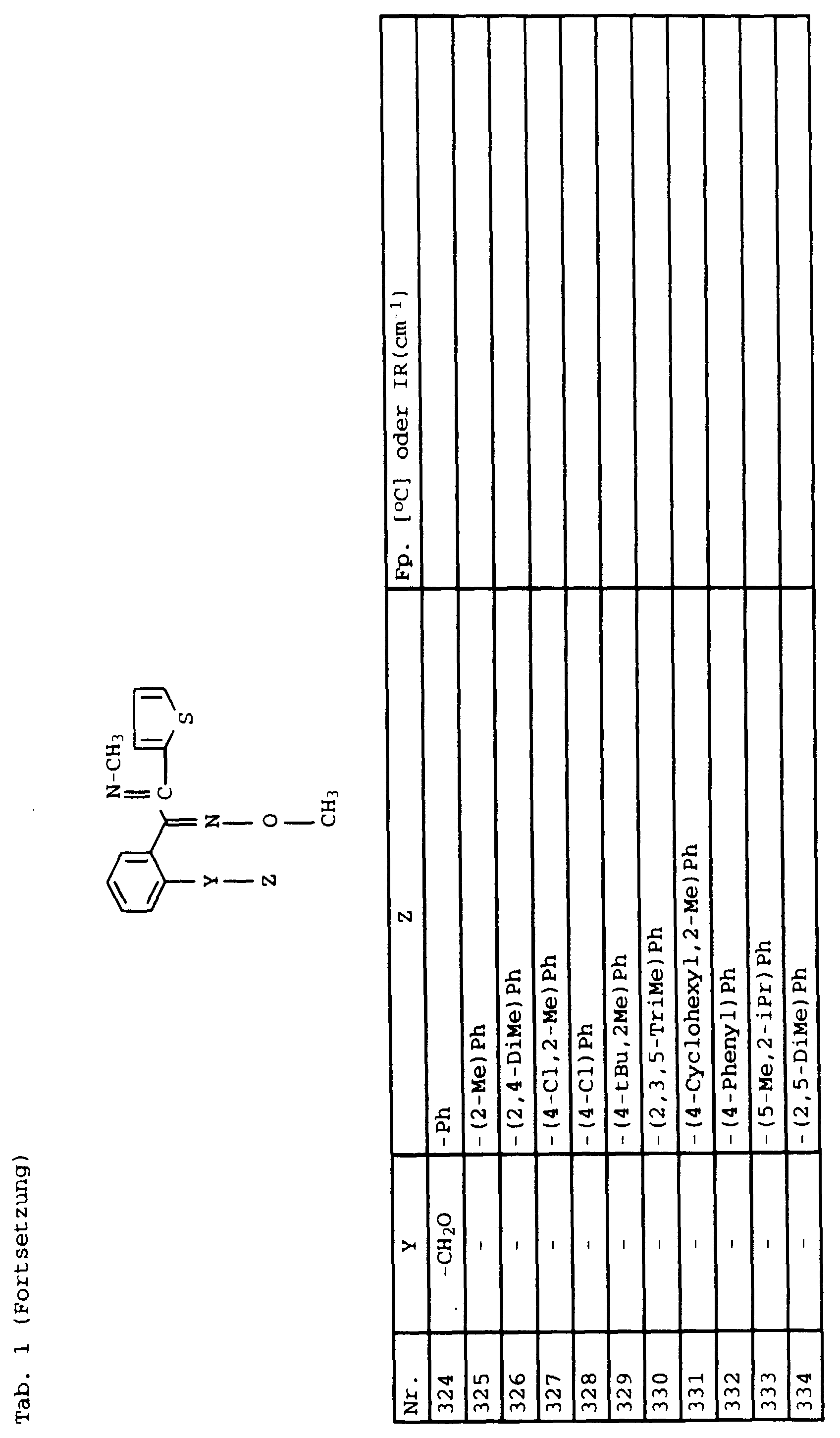

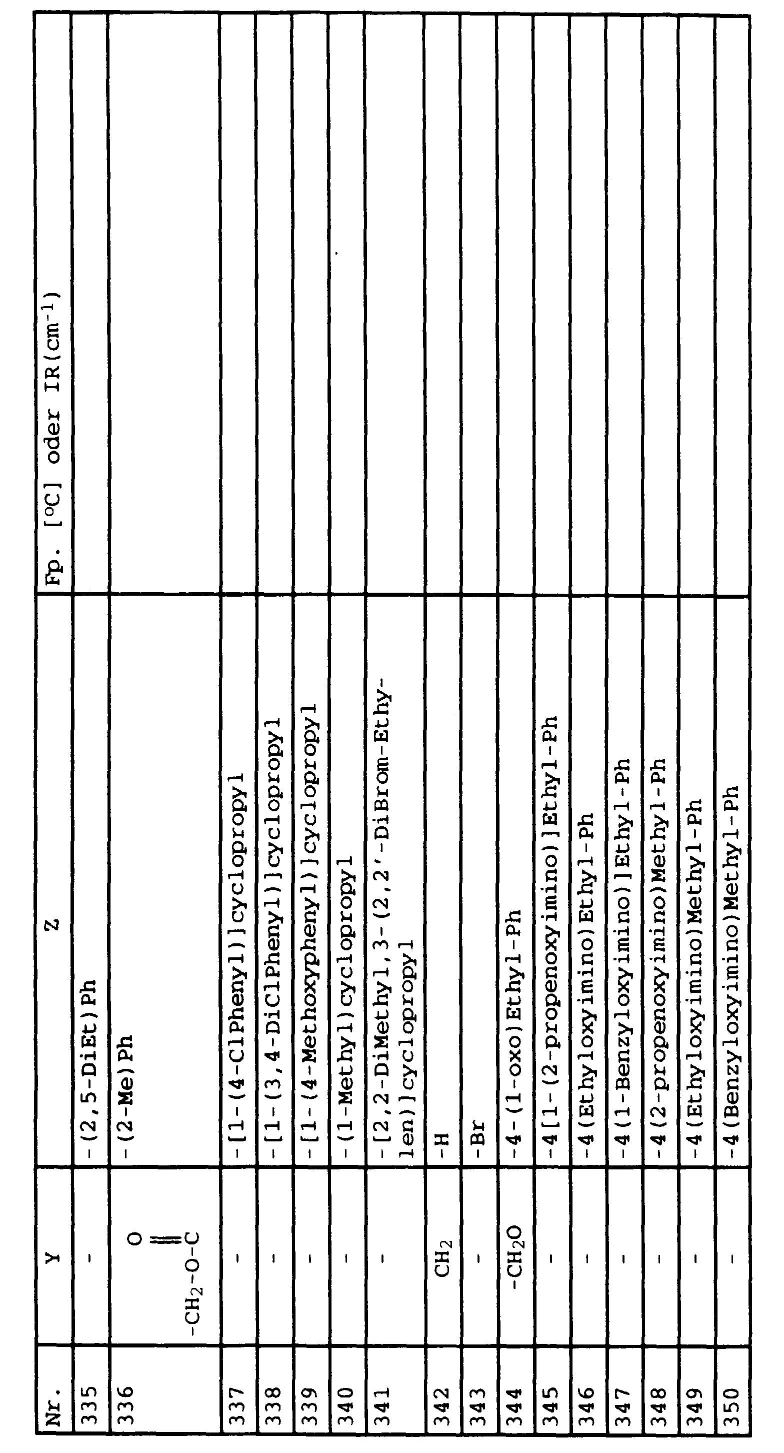

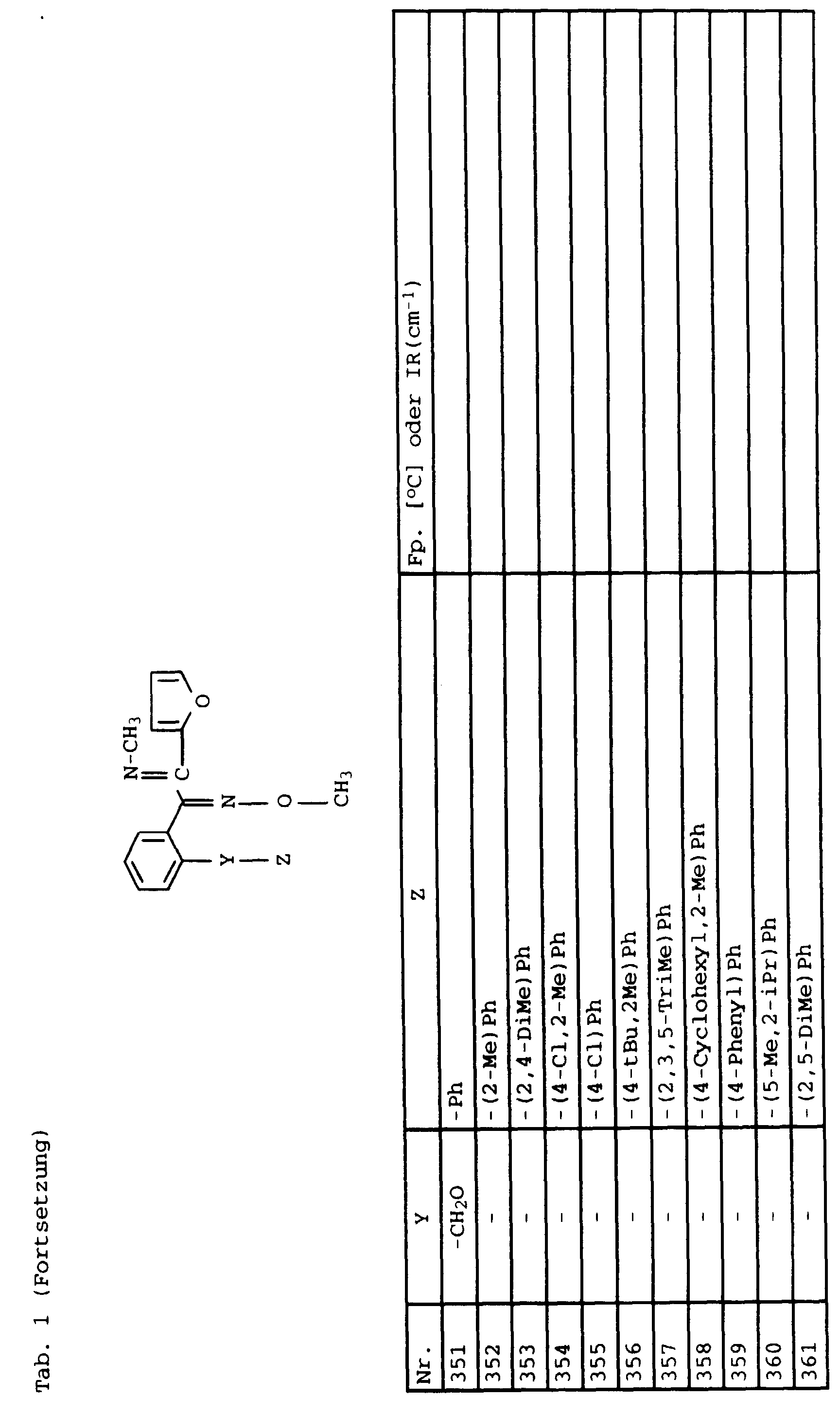

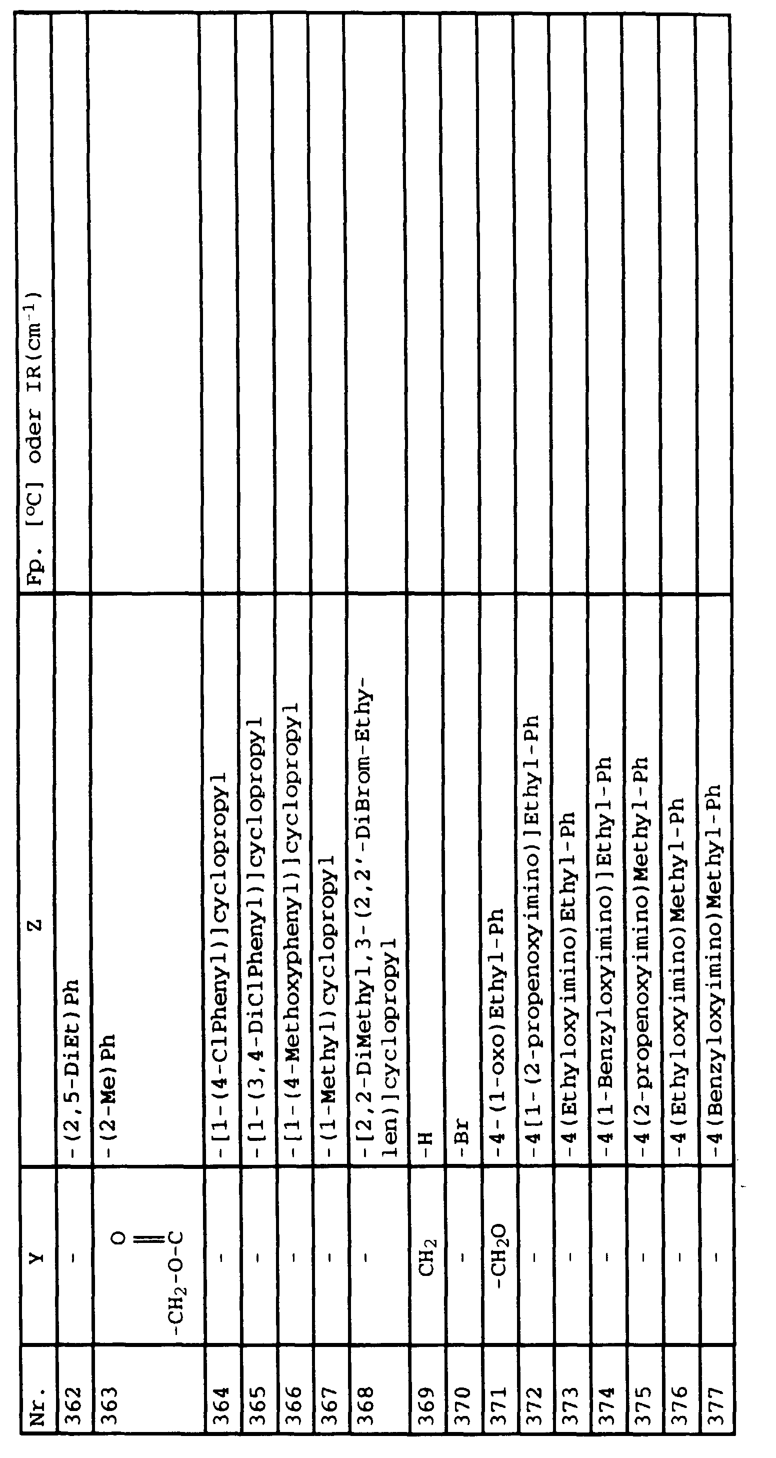

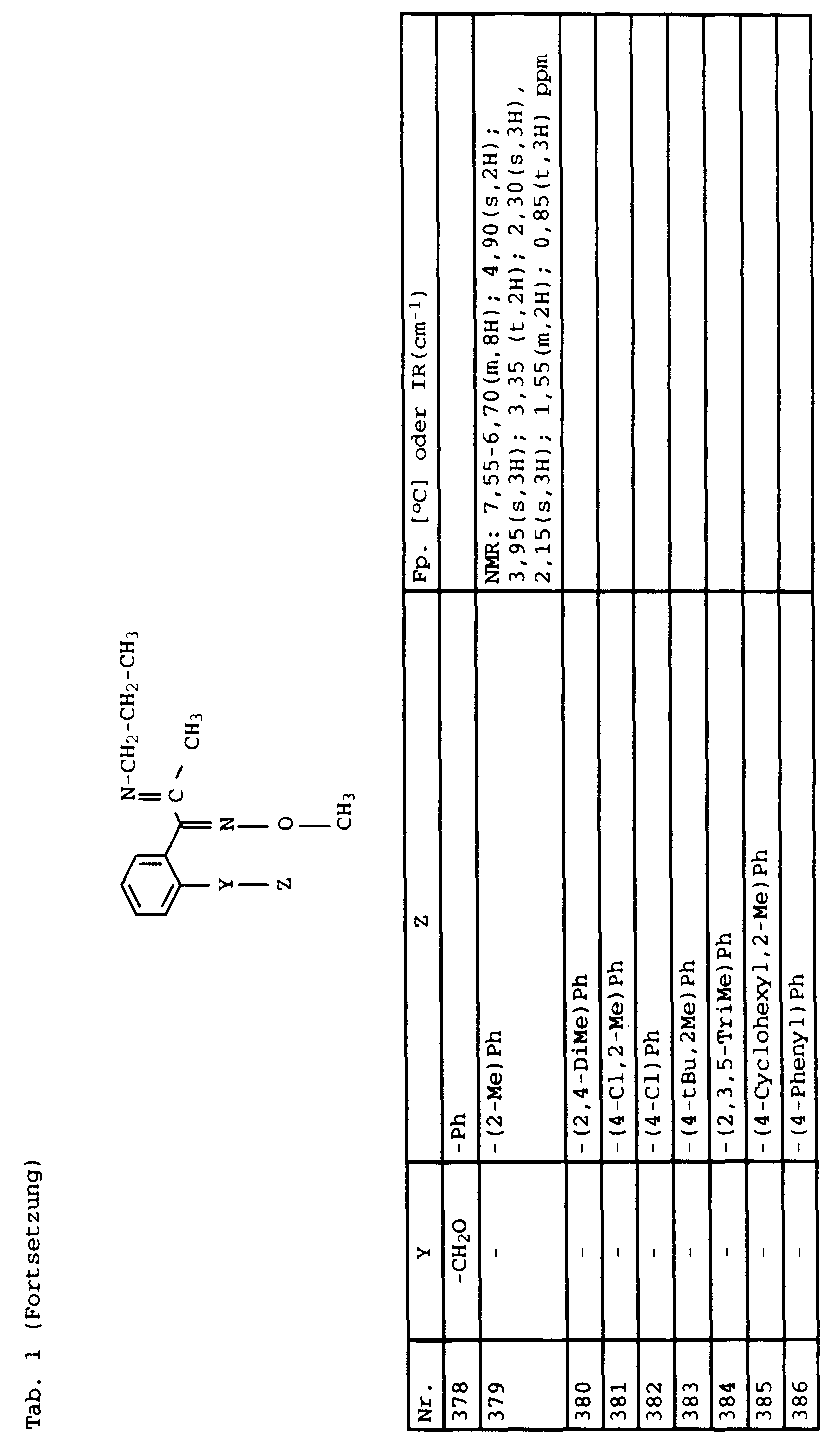

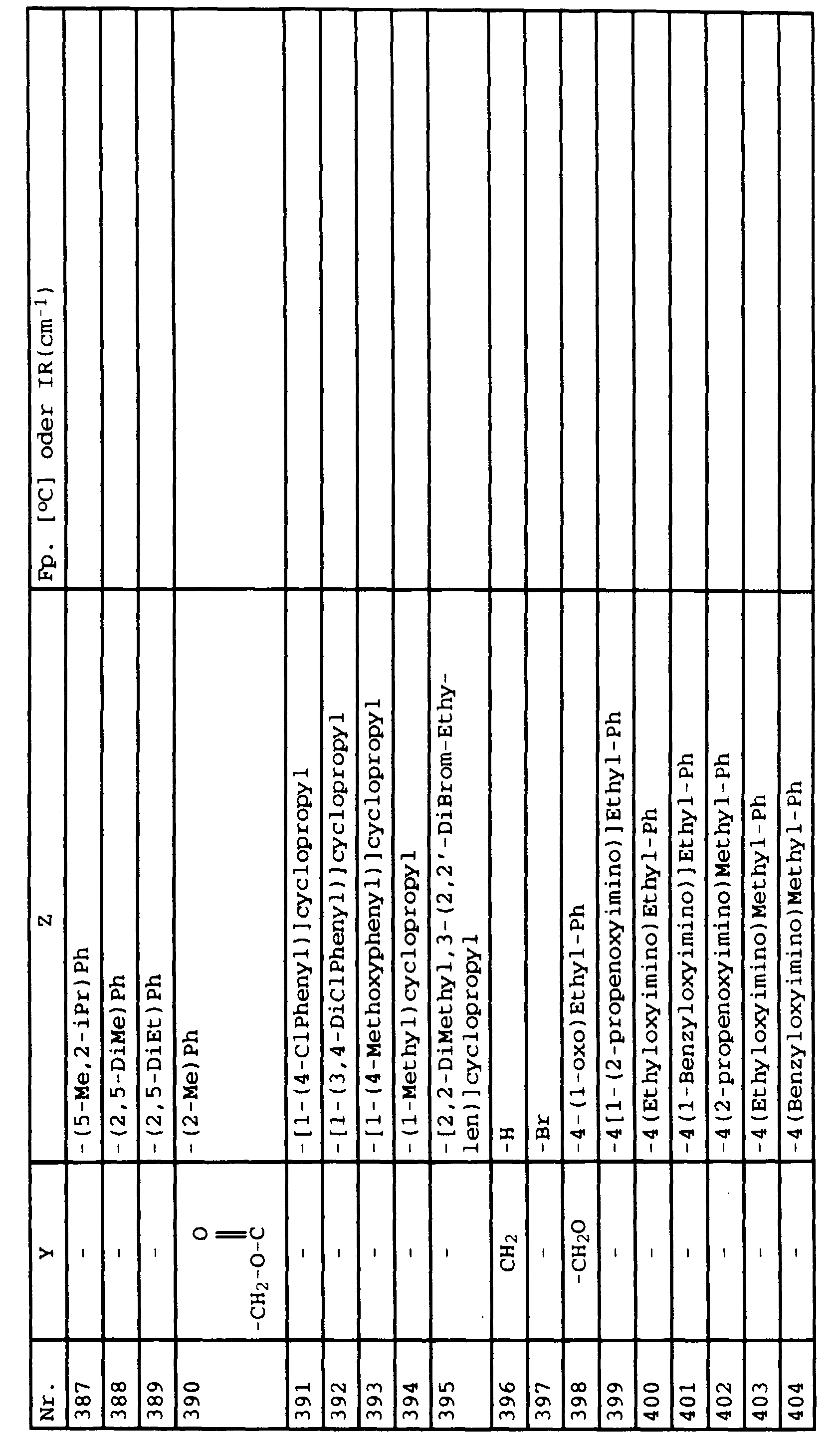

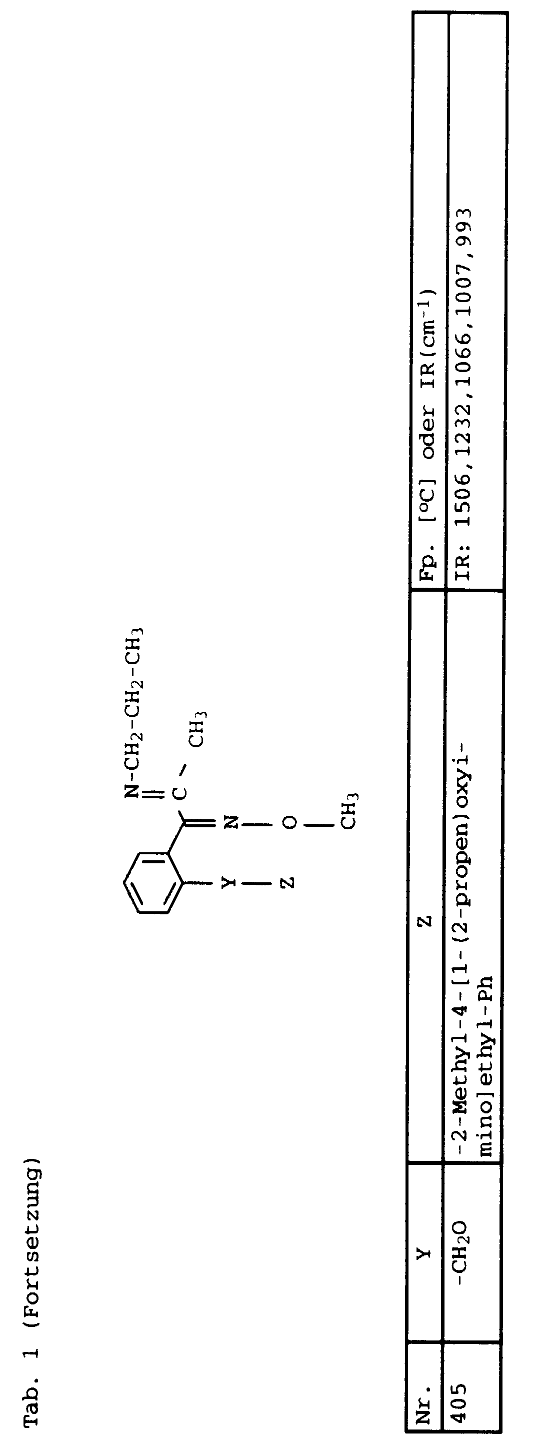

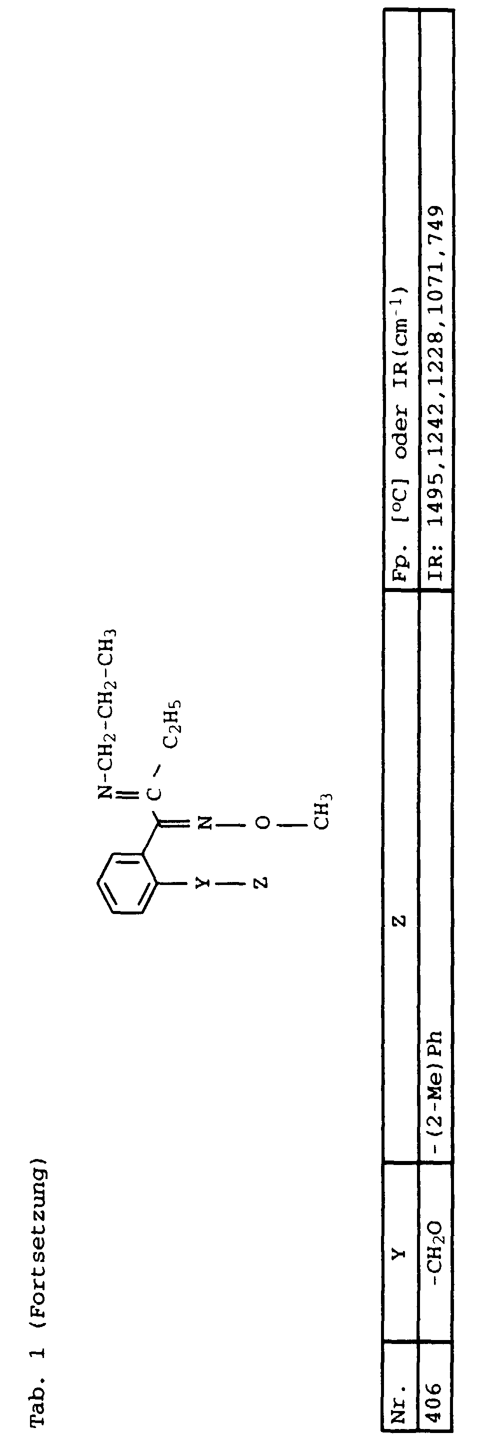

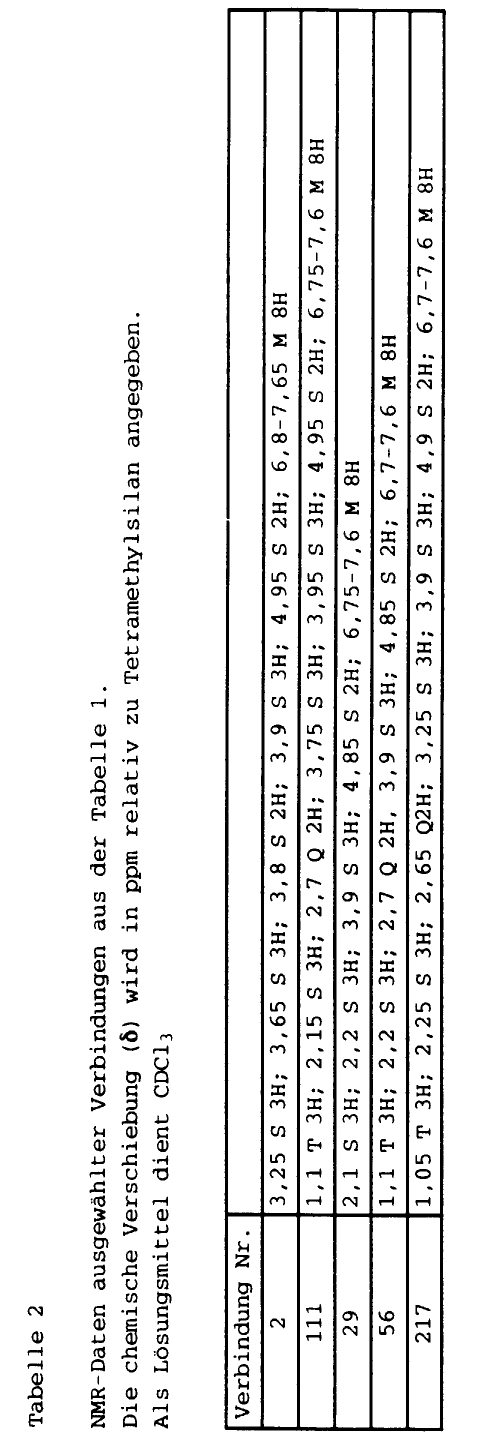

Die neuen Oximether eignen sich als Fungizide.

Die erfindungsgemäßen fungiziden Oximether bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon, Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Oximether zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Oximether zur Bekämpfung folgender Pflanzenkrankheiten:
Erysiphe graminis (echter Mehltau) in Getreide, Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

### Anwendungsbeispiel

Als Vergleichswirkstoff wurde 1-Methoxyimino-1-(2-phenoxymethyl)-phenylessigsäuremethylester (A) - bekannt aus EP 253 213 - benutzt.

### Anwendungsbeispiel

### Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis zeigt, daß die Wirkstoffe 164, 190 und 217 bei der Anwendung als 0,0015 %ige (Gew.-%) Spritzbrühe eine bessere fungizide Wirkung zeigen (95 %) als der bekannte Vergleichswirkstoff A (75 %).

## Patentansprüche

1. Oximether der Formel I 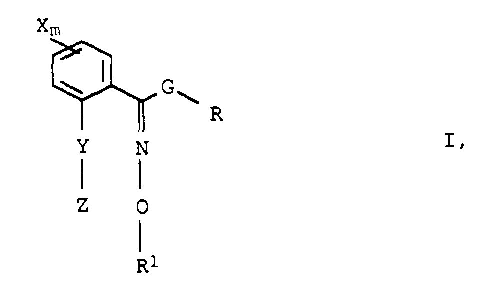 in der
G C=W, bedeutet,
W N-R⁵ bedeutet,
R gegebenenfalls substituiertes C₃-C₈-Cycloalkyl, gegebenenfalls substituiertes C₂-C₈-Alkenyl, gegebenenfalls substituiertes C₂-C₈-Alkinyl, gegebenenfalls substituiertes Phenyl, gegebenenfalls substituiertes Hetaryl oder 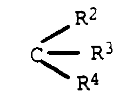 bedeutet,
R¹ Wasserstoff, C₁-C₈-Alkyl bedeutet,
R² Wasserstoff, C₁-C₈-Alkyl bedeutet, Arylalkyl, Hetarylalkyl, Alkoxyalkyl, Aryloxyalkyl, Aryl, Hetaryl, bedeutet,
R³ Wasserstoff, Methyl, Cyano, -COO-A bedeutet,
A Wasserstoff, C₁-C₈-Alkyl bedeutet,
oder
für den Fall, daß R³ den Rest -COO-C₁-C₈-Alkyl bedeutet, R² und R³ zusammen mit dem C, dessen Substituenten sie sind, einen 5- bis 8-gliedrigen Lactonring bedeuten,
R⁴ Wasserstoff, C₁-C₈-Alkyl, Arylalkyl, Hetarylalkyl, Alkoxyalkyl, Aryloxyalkyl, Aryl, Hetaryl bedeutet,
oder
R² und R⁴ zusammen mit dem C, dessen Substituenten sie sind, einen C₃-C₈-Cycloalkylring bedeuten,
R⁵ Wasserstoff, C₁-C₈-Alkyl, C₁-C₄-Arylalkyl, C₃-C₆-Cycloalkyl, Hydroxy, C₁-C₄-Alkyloxy, NH₂, C₁-C₄-Alkylamino, C₁-C₄-Dialkylamino bedeutet,
Y gegebenenfalls substituiertes C₁-C₈-Alkylen, gegebenenfalls substituiertes C₂-C₈-Alkenylen, C₂-C₈-Alkinylen, O, S(O)ₘ (m = 0, 1, 2), N, das substituiert ist durch gegebenenfalls substituiertes C₁-C₈-Alkyl; Oxycarbonyl, Carbonyloxy, Oxycarbonyl-C₁-C₈-alkyl, Carbonyloxy-C₁-C₈-alkyl, C₂-C₈-Oxyalkyloxy, C₂-C₈-Oxyalkenyl, C₁-C₈-Alkyloxy, Oxy-C₁-C₄-alkyl, C₁-C₈-Thioalkyl, Azo, gegebenenfalls C₁-C₈-alkylsubstituiertes Carbonylamino, gegebenenfalls C₁-C₈-alkylsubstituiertes Aminocarbonyl, gegebenenfalls C₁-C₈-alkylsubstituiertes Aminocarbonyloxy, Oxyimino, gegebenenfalls substituiertes Iminooxyalkyl bedeutet,
Z C₁-C₁₈-Alkyl, C₂-C₁₈-Alkenyl, C₃-C₁₈-Alkinyl, Aryl, Aryl-C₁-C₁₀-Alkyl, Aryl-C₂-C₁₀-Alkenyl, Aryloxy-C₁-C₁₀-Alkyl, C₁-C₄-Alkoxy-C₁-C₁₀-alkyl, C₁-C₁₀-Halogenalkyl, C₁-C₄-Alkoxycarbonyl, Hetaryl bedeutet,
Z bedeutet für den Fall, daß Y nicht Oxyalkyl, Oxyalkyloxy, Thialkyl, -O-N=C-, -N=N-, Oxycarbonyl, Oxycarbonyloxy, O oder S ist, zusätzlich noch Aryloxy, Aryloxy-C₁-C₁₀-alkoxy, Aryl-C₁-C₁₀-alkoxy, C₁-C₁₀-Alkoxy oder Hetaryloxy,
Z bedeutet für den Fall, daß Y nicht Oxyalkyl, Oxyalkyloxy, -O-N=C-, -N=N-, Oxycarbonyl, Oxycarbonylalkyl oder O ist, zusätzlich noch C₁-C₁₈-Alkylthio oder Arylthio,
wobei die für Z genannten Reste gegebenenfalls substituiert sind durch Halogen, Cyano, Nitro, C₁-C₄-Alkyl, C₂-C₄-Alkenyl, C₁-C₄-Alkoxy, C₁-C₄-Halogenalkyl, C₁-C₄-Alkoxy-C₁-C₄-alkyl, C₁-C₄-Dialkoxy-C₁-C₄-Alkyl, gegebenenfalls substituiertes O-C₁-C₁₀-Alkyloxyimino, gegebenenfalls substituiertes O-C₂-C₁₀-Alkenyloxyimino, gegebenenfalls substituiertes O-Aralkyloxyimino, gegebenenfalls substituiertes C₂-C₁₀-Alkanoyl, gegebenenfalls substituiertes Formyl, C₂-C₄-Halogenalkenyl, C₁-C₄-Alkoxy-carbonyl, gegebenenfalls substituiertes Phenyl, gegebenenfalls substituierten fünfgliedrigen Heterocyclus, der ein bis vier gleiche oder verschiedene Heteroatome nämlich Stickstoff, Sauerstoff oder Schwefel enthält, bedeutet, in dem zwei benachbarte Substituenten einen gegebenenfalls substituierten aromatischen oder heteroaromatischen Ring bilden können,
X gleich oder verschieden ist und Wasserstoff, Halogen, Nitro, gegebenenfalls substituiertes C₁-C₄-Alkyl, gegebenenfalls substituiertes C₁-C₄-Alkoxy oder gegebenenfalls substituiertes C₁-C₄-Halogenalkyl bedeutet und
m eine ganze Zahl zwischen 1 und 4 bedeutet

2. Oximether der Formel I gemäß Anspruch 1, in der G C=NCH₃, R CR²R³R⁴, R¹ Methyl, R² und R³ Wasserstoff, R⁴ Methyl, Y Oxymethylen, Z 2-Methylphenyl bedeutet.

3. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge einer Verbindung der Formel I gemäß Anspruch 1 behandelt.

4. Fungizid, enthaltend einen inerten Trägerstoff und eine fungizid wirksame Menge einer Verbindung der Formel I gemäß Anspruch 1.

5. Oximether nach Anspruch 1 in denen
R¹ C₁-C₈-Alkyl bedeutet, und
Z C₁-C₁₈-Alkyl, C₂-C₁₈-Alkenyl, C₃-C₁₈-Alkinyl, Aryl, Aryl-C₁-C₁₀-Alkyl, Aryl-C₂-C₁₀-Alkenyl, Aryloxy-C₁-C₁₀-Alkyl, C₁-C₄-Alkoxy-C₁-C₁₀-alkyl, C₁-C₁₀-Halogenalkyl, C₁-C₄-Alkoxycarbonyl, Hetaryl bedeutet.

## Claims

1. An oxime ether of the formula I 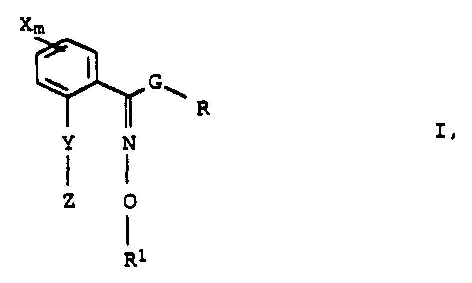 where
G is C=W,
W is N-R⁵,
R is unsubstituted or substituted C₃-C₈-cycloalkyl, unsubstituted or substituted C₂-C₈-alkenyl, unsubstituted or substituted C₂-C₈-alkynyl, unsubstituted or substituted phenyl, unsubstituted or substituted hetaryl or 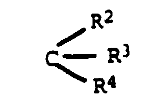
R¹ is hydrogen or C₁-C₈-alkyl,
R² is hydrogen, C₁-C₈-alkyl, arylalkyl, hetarylalkyl, alkoxyalkyl, aryloxyalkyl, aryl or hetaryl,
R³ is hydrogen, methyl, cyano or -COO-A,
A is hydrogen, C₁-C₈-alkyl,
or
in the case where R³ is the radical -COO-C₁-C₈-alkyl, R² and R³, together with the C whose substituents they are, are a 5- to 8-membered lactone ring,
R⁴ is hydrogen, C₁-C₈-alkyl, arylalkyl, hetarylalkyl, alkoxyalkyl, aryloxyalkyl, aryl or hetaryl,
or
R² and R⁴, together with the C whose substituents they are, are a C₃-C₈-cycloalkyl ring,
R⁵ is hydrogen, C₁-C₈-alkyl, C₁-C₄-arylalkyl, C₃-C₆-cycloalkyl, hydroxyl, C₁-C₄-alkoxy, NH₂, C₁-C₄-alkylamino or C₁-C₄-dialkylamino,
Y is unsubstituted or substituted C₁-C₈-alkylene, unsubstituted or substituted C₂-C₈-alkenylene, C₂-C₈-alkynylene, O, S(O)ₘ (m = 0, 1 or 2), N which is substituted by unsubstituted or substituted C₁-C₈-alkyl; oxycarbonyl, carbonyloxy, oxycarbonyl-C₁-C₈-alkyl, carbonyloxy-C₁-C₈-alkyl, C₂-C₈-oxyalkoxy, C₂-C₈-oxyalkenyl, C₁-C₈-alkoxy, oxy-C₁-C₄-alkyl, C₁-C₈-thioalkyl, azo, unsubstituted or C₁-C₈-alkyl-substituted carbonylamino, unsubstituted or C₁-C₈-alkyl-substituted aminocarbonyl, unsubstituted or C₁-C₈-alkyl-substituted aminocarbonyloxy, oxyimino or unsubstituted or substituted iminooxyalkyl,
Z is C₁-C₁₈-alkyl, C₂-C₁₈-alkenyl, C₃-C₁₈-alkynyl, aryl, aryl-C₁-C₁₀-alkyl, aryl-C₂-C₁₈-alkenyl, aryloxy-C₁-C₁₀-alkyl, C₁-C₄-alkoxy-C₁-C₁₀-alkyl, C₁-C₁₀-haloalkyl, C₁-C₄-alkoxycarbonyl or hetaryl,
Z, in the case where Y is not oxyalkyl, oxyalkoxy, thioalkyl, -O-N=C-, -N=N-, oxycarbonyl, oxycarbonyloxy, O or S, is additionally aryloxy, aryloxy-C₁-C₁₀-alkoxy, aryl-C₁-C₁₀-alkoxy, C₁-C₁₀-alkoxy or hetaryloxy,
Z, in the case where Y is not oxyalkyl, oxyalkoxy, -O-N=C-, -N=N-, oxycarbonyl, oxycarbonylalkyl or O, is additionally C₁-C₁₈-alkylthio or arylthio,
the radicals mentioned for Z being unsubstituted or substituted by halogen, cyano, nitro, C₁-C₄-alkyl, C₂-C₄-alkenyl, C₁-C₄-alkoxy, C₁-C₄-haloalkyl, C₁-C₄-alkoxy-C₁-C₄-alkyl, C₁-C₄-dialkoxy-C₁-C₄-alkyl, unsubstituted or substituted O-C₁-C₁₀-alkoxyimino, unsubstituted or substituted O-C₂-C₁₀-alkenyloxyimino, unsubstituted or substituted O-aralkoxyimino, unsubstituted or substituted C₂-C₁₀-alkanoyl, unsubstituted or substituted formyl, C₂-C₄-haloalkenyl, C₁-C₄-alkoxycarbonyl, unsubstituted or substituted phenyl, an unsubstituted or substituted 5-membered heterocycle which contains one to four identical or different heteroatoms, namely nitrogen, oxygen or sulfur, in which two adjacent substituents can form an unsubstituted or substituted aromatic or heteroaromatic ring,
X is identical or different and is hydrogen, halogen, nitro, unsubstituted or substituted C₁-C₄-alkyl, unsubstituted or substituted C₁-C₄-alkoxy or unsubstituted or substituted C₁-C₄-haloalkyl and
m is an integer from 1 to 4.

2. An oxime ether of the formula I as claimed in claim 1, where G is C=NCH₃, R is CR²R³R⁴, R¹ is methyl, R² and R³ are hydrogen, R⁴ is methyl, Y is oxymethylene and Z is 2-methylphenyl.

3. A method of controlling fungi, which comprises treating the fungi or the materials, plants, seed or the soil threatened by fungal attack with a fungicidally active amount of a compound of the formula I as claimed in claim 1.

4. A fungicide comprising an inert carrier and a fungicidally active amount of a compound of the formula I as claimed in claim 1.

5. An oxime ether as claimed in claim 1 where
R¹ is C₁-C₈-alkyl, and
Z is C₁-C₁₈-alkyl, C₂-C₁₈-alkenyl, C₃-C₁₈-alkynyl, aryl, aryl-C₁-C₁₀-alkyl, aryl-C₂-C₁₀-alkenyl, aryloxy-C₁-C₁₀-alkyl, C₁-C₄-alkoxy-C₁-C₁₀-alkyl, C₁-C₁₀-haloalkyl, C₁-C₄-alkoxycarbonyl or hetaryl.

## Revendications

1. Ethers d'oximes de formule I 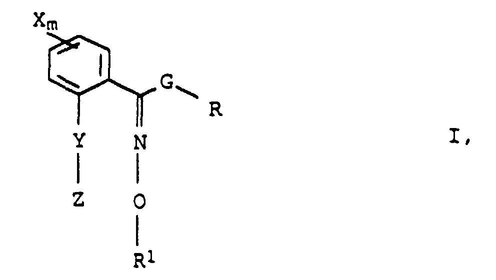 dans laquelle
G représente C=W
W représente N-R⁵,
R représente un groupe cycloalkyle en C3-C8 éventuellement substitué, alcényle en C2-C8 éventuellement substitué, alcynyle en C2-C8 éventuellement substitué, phényle éventuellement substitué, hétéroaryle éventuellement substitué ou 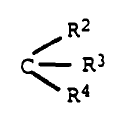
R¹ représente l'hydrogène, un groupe alkyle en C1-C8,
R² représente l'hydrogène, un groupe alkyle en C1-C8, arylalkyle, hétéroarylalkyle, alcoxyalkyle, aryloxyalkyle, aryle, hétéroaryle,
R³ représente l'hydrogène, un groupe méthyle, cyano, -COO-A,
A représente l'hydrogène, un groupe alkyle en C1-C8,
ou bien,
lorsque R³ représente un groupe -COO-alkyle en C1-C8, R² et R³ forment ensemble et avec le C dont ils sont substituants, un cycle lactone de 5 à 8 chaînons,
R⁴ représente l'hydrogène, un groupe alkyle en C1-C8, arylalkyle, hétéroarylalkyle, alcoxyalkyle, aryloxyalkyle, aryle, hétéroaryle,
ou bien
R² et R⁴ forment ensemble et avec le C dont ils sont substituants, un noyau cycloalkyle en C3-C8,
R⁵ représente l'hydrogène, un groupe alkyle en C1-C8, aryl-alkyle en C1-C4, cycloalkyle en C3-C6, hydroxy, alkyloxy en C1-C4, NH₂, alkylamino en C1-C4, dialkylamino en C1-C4,
Y représente un groupe alkylène en C1-C8 éventuellement substitué, alcénylène en C2-C8 éventuellement substitué, alcynylène en C2-C8, O, S(O)ₘ (m = 0, 1, 2), N éventuellement substitué par un groupe alkyle en C1-C8 ; un groupe oxycarbonyle, carbonyloxy, oxycarbonylalkyle en C1-C8, carbonyloxy-alkyle en C1-C8, oxyalkyloxy en C2-C8, oxyalcényle en C2-C8, alkyloxy en C1-C8, oxyalkyle en C1-C4, thioalkyle en C1-C8, azo, carbonylamino éventuellement substitué par un groupe alkyle en C1-C8, aminocarbonyle éventuellement substitué par un groupe alkyle en C1-C8, aminocarbonyloxy éventuellement substitué par un groupe alkyle en C1-C8, oxyimino, iminooxyalkyle éventuellement substitué,
Z représente un groupe alkyle en C1-C8, alcényle en C2-C18, alcynyle en C3-C18, aryle, aryl-alkyle en C1-C10, aryl-alcényle en C2-C10, aryloxy-alkyle en C1-C10, (alcoxy en C1-C4)-alkyle en C1-C10, halogénoalkyle en C1-C10, (alcoxy en C1-C4)carbonyle, hétéroaryle,
Z, lorsque Y ne représente pas un groupe oxyalkyle, oxyalkyloxy, thioalkyle, -O-N=C-, -N=N-, oxycarbonyle, oxycarbonyloxy, O ou S, peut en outre représenter un groupe aryloxy, aryloxy-alcoxy en C1-C10, aryl-alcoxy en C1-C10, alcoxy en C1-C10 ou hétéroaryle, Z, lorsque Y ne représente pas un groupe oxyalkyle, oxyalkyloxy, -O-N=C-, -N=N-, oxycarbonyle, oxycarbonylalkyle ou 0, peut en outre représenter un groupe alkylthio en C1-C18 ou arylthio,
les radicaux mentionnés en référence à Z pouvant le cas échéant être substitués par des halogènes, des groupes cyano, nitro, alkyle en C1-C4, alcényle en C2-C4, alcoxy en C1-C4, halogénoalkyle en C1-C4, (alcoxy en C1-C4)-alkyle en C1-C4, (dialcoxy en C1-C4)-alkyle en C1-C4, O-alkyloximino en C1-C10 éventuellement substitué, O-alcényloxyimino en C2-C10 éventuellement substitué, O-aralkyloxyimino éventuellement substitué, alcanoyle en C2-C 10 éventuellement substitué, formyle éventuellement substitué, halogénoalcényle en C2-C4, (alcoxy en C1-C4)-carbonyle, phényle éventuellement substitué, hétérocyclyle à cinq chaînons éventuellement substitué, qui contient un à quatre hétéroatomes identiques ou différents choisis parmi l'azote, l'oxygène ou le soufre et dans lequel deux substituants voisins peuvent former un cycle aromatique ou hétéroaromatique éventuellement substitué,
les symboles X, ayant des significations identiques ou différentes, représentent chacun l'hydrogène, un halogène, un groupe nitro, un groupe alkyle en C1-C4 éventuellement substitué, alcoxy en C1-C4 éventuellement substitué ou hélogénoalkyle en C1-C4 éventuellement substitué et
m est un nombre entier allant de 1 à 4.

2. Ethers d'oximes de formule I de la revendication 1, dans laquelle G représente C=NCH₃, R représente CR²R³R⁴, R¹ représente un groupe méthyle, R² et R³ l'hydrogène, R⁴ un groupe méthyle, Y un groupe oxyméthylène et Z un groupe 2-méthylphényle.

3. Procédé pour combattre les mycètes, caractérisé par le fait que l'on traite les mycètes ou les matières, végétaux, semences ou sols menacés d'une attaque par les mycètes par une quantité fongicide efficace d'un composé de formule I de la revendication 1.

4. Fongicide contenant un véhicule inerte et une quantité fongicide efficace d'un composé de formule I de la revendication 1.

5. Ethers d'oximes selon la revendication 1, pour lesquels
R¹ représente un groupe alkyle en C1-C8 et
Z représente un groupe alkyle en C1-C18, alcényle en C2-C18, alcynyle en C3-C18, aryle, aryl-alkyle en C1-C10, aryl-alcényle en C2-C10, aryloxy-alkyle en C1-C10, (alcoxy en C1-C4)-alkyle en C1-C10, halogénoalkyle en C1-C10, (alcoxy en C1-C4)carbonyle, hétéroaryle.
